(19) Europäisches Patentamt / European Patent Office / Office européen des brevets

(11) EP 3 290 429 A1

(12) **EUROPEAN PATENT APPLICATION**
published in accordance with Art. 153(4) EPC

(43) Date of publication:
**07.03.2018 Bulletin 2018/10**

(21) Application number: **16785828.1**

(22) Date of filing: **13.04.2016**

(51) Int Cl.:
***C07J 9/00*** *(2006.01)* ***C07J 31/00*** *(2006.01)*
***C07J 41/00*** *(2006.01)* ***C07J 51/00*** *(2006.01)*
***C07J 43/00*** *(2006.01)* ***C07J 75/00*** *(2006.01)*
***A61K 31/58*** *(2006.01)* ***A61K 31/575*** *(2006.01)*
***A61P 9/00*** *(2006.01)* ***A61P 3/06*** *(2006.01)*
***A61P 1/16*** *(2006.01)* ***A61P 1/00*** *(2006.01)*

(86) International application number:
**PCT/CN2016/079167**

(87) International publication number:
**WO 2016/173397 (03.11.2016 Gazette 2016/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **28.04.2015 CN 201510204748**

(71) Applicants:
- **Jiangsu Hansoh Pharmaceutical Group Co., Ltd. Jiangsu 222047 (CN)**
- **Shanghai Hansoh Biomedical Co., Ltd. Shanghai 201203 (CN)**

(72) Inventors:
- **LU, Aifeng Lianyungang, Jiangsu 222047 (CN)**
- **ZHONG, Huijuan Lianyungang, Jiangsu 222047 (CN)**
- **LI, Chenghai Shanghai 201203 (CN)**
- **BAO, Rudi Shanghai 201203 (CN)**
- **LV, Aifeng Lianyungang Jiangsu 222047 (CN)**
- **ZHONG, Huijuan Lianyungang Jiangsu 222047 (CN)**

(74) Representative: **Beresford Crump LLP**
**16 High Holborn**
**London**
**WC1V 6BX (GB)**

# (54) CHOLIC ACID DERIVATIVE, AND PREPARATION METHOD AND MEDICAL USE THEREOF

(57) The present invention relates to a cholic acid derivative, and a preparation method and a medical use thereof. In particular, the present invention relates to a cholic acid derivative with the structure of formula (I), a stereoisomer, or a pharmaceutically acceptable salt thereof. The series of compounds can be used to treat FXR mediated diseases, comprising cardiovascular disease, atherosclerosis, arteriosclerosis, hypercholesterolemia, hyperlipidemia and chronic hepatitis diseases, chronic liver diseases, gastrointestinal diseases, nephrosis, heart vascular diseases, metabolic diseases, cancer (for example colorectal cancer) or neurological signs, such as strokes and other diseases, with a wide range of medical applications, and are expected to develop into a new generation of FXR agonist.

EP 3 290 429 A1

## Description

### FIELD OF THE INVENTION

The present invention belongs to the field of pharmaceutical synthesis, and specifically relates to a cholic acid derivative, and the preparation method and use thereof.

### BACKGROUND OF THE INVENTION

The farnesyl derivative X receptor (FXR) is a member of the hormone nuclear receptor superfamily, which is mainly expressed in liver, small intestine, kidney and adrenal gland, and is less expressed in adipose tissue and heart. It is named as FXR because farnesol is thought as its ligand initially. When the FXR ligand directly binds to the ligand-binding region(LBD) of carboxy terminal of FXR, the conformation of the nuclear receptor changes and forms a heterodimer with the retinal derivative receptor (RXR), which is ultimately integrated with the specific FXR DNA response element of target gene to regulate the transcription of the target gene, involved in the regulation of sugar and lipid metabolism. FXR thus is an important energy regulator. Primary bile acid chenodeoxycholic acid is the most effective ligand for FXR, and secondary bile acid lithocholic acid and deoxycholic acid can also activate FXR. There are also synthetic FXR ligands (such as 6-ECDCA, GW4064, etc.), which is several times stronger than natural ligands in binding FXR. The main target genes of FXR include bile salt export pump (BSEP), bile acid binding protein (IBABP) and small heterodimeric partner receptor (SHP), etc. FXR regulates the expression of these genes by reacting with FXR reaction elements (FXRE) on these gene promoters. However, there is no typical FXR binding response sequences in the promoter sequence of certain major FXR regulatory genes, such as cholesterol 7$\alpha$ hydroxylase (CYP7$\alpha$1), so FXR blocks the transcription of CYP7$\alpha$1 and other LRH-1 target genes by indirectly inducing the expression of transcriptional repressor SHP, which then forms inhibition complexes with the liver receptor homologue (LRH-1) of CYP7$\alpha$1 promoter. FXR plays an important role in bile acid and cholesterol metabolism, which will make its relationship between liver-related diseases attracted more and more attention. Finding a new FXR agonist has become a hot research topic in the treatment of liver diseases including cholestasis syndrome. As a treatment for primary biliary cirrhosis (PBC), 6-ethyl chenodeoxycholic acid (obeticholic acid) has been completed in Phase III Clinical trials, and will be list in the market as early as 2015. This compound also shows satisfactory treatment effect in non-alcoholic steatohepatitis. FXR agonists either can increase bile acid-dependent bile flow by stimulating bile salt export pump (BSEP), or can reduce cholestasis by stimulating MRP2 to increase non-bile acid-dependent bile flow. FXR is expected to be a target for screening new drug which can treat other metabolic diseases, including cholestatic diseases and nonalcoholic steatohepatitis and the like. Furthermore, the current studies have shown that FXR agonists may be valuable in the treatment and research of diseases including atherosclerosis, cholestatic diseases caused by bile acid disorder, liver fibrosis, cirrhosis, cancer and the like (please refer to table 1).

Table 1. The diseases can be benefited from FXR agonists

- arteriosclerosis
- bile acid disorders
  - benign intrahepatic cholestasis
  - progressive familial intrahepatic cholestasis (type 1, 2 and 3)
  - primary biliary cirrhosis
  - primary biliary cirrhosis, primary sclerosing cholangitis
- cancer
- cholesterol gallstone
- diabetes
- dyslipidemia
- fibrosis diseases
  - chronic hepatitis (type B and C type)
  - non-viral hepatitis
- inflammatory bowel disease
- alteration of intestinal flora
- liver transplantation
- non-alcoholic fattv liver disease and non-alcoholic steatohepatitis

## SUMMARY OF THE INVENTION

During the course of research, the inventors found a series of cholic acid derivatives as represented by formula (I). These compounds have a significant agonistic effect on FXR activity, and can be used in the treatment of FXR mediated diseases comprising cardiovascular disease, atherosclerosis, arteriosclerosis, hypercholesterolemia, hyperlipidemia and chronic hepatitis diseases, chronic liver diseases, gastrointestinal diseases, nephrosis, heart vascular diseases, metabolic diseases, cancer (for example colorectal cancer) or neurological signs, such as strokes and other diseases, with a wide range of medical applications, and are expected to be developed into a new generation of FXR agonist.

In one aspect, the present invention provides a cholic acid derivatives of formula (I), a stereoisomer or a pharmaceutically acceptable salt thereof:

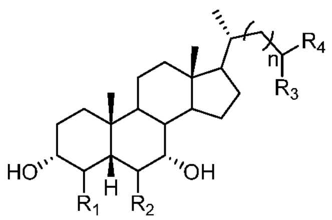

**(I)**

wherein,

$R_1$, $R_2$ and $R_3$ are each selected from the group consisting of hydrogen, fluorine, bromine, trifluoromethyl and $C_{1-8}$ alkyl;
$R_4$ is selected from the group consisting of:

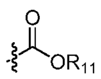 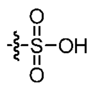 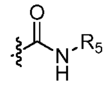 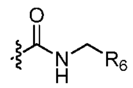

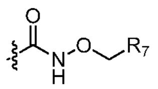 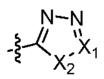 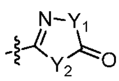 and 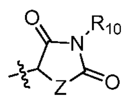 ;

$X_1$ is selected from the group consisting of nitrogen and $CR_8$;
$X_2$ is selected from the group consisting of $CR_8R_9$, $NR_{10}$, oxygen and sulfur atom;
$Y_1$ and $Y_2$ are each independently selected from the group consisting of $CR_8R_9$, $NR_{10}$, oxygen and sulfur atom;
Z is selected from the group consisting of $CR_8R_9$, $NR_{10}$, oxygen and sulfur atom;
$R_5$ is selected from the group consisting of hydrogen, hydroxy, cyano, amino, $C_{1-8}$ alkoxy and $SO_2R_{11}$;
$R_6$ is selected from the group consisting of $SO_2R_{11}$, $SO_3R_{11}$, $C(O)OR_{11}$, $C(O)R_{12}$ and $C_{0-4}$-$P(O)R_{13}R_{14}$;
$R_7$ is selected from the group consisting of hydroxyl, $C_{1-8}$ alkyl and halo$C_{1-8}$ alkyl substituted with hydroxy;
$R_8$ and $R_9$ are each independently selected from the group consisting of hydrogen, deuterium, halogen, hydroxy, cyano, nitro, $C_{1-8}$ alkyl, $C_{2-8}$ alkenyl, $C_{2-8}$ alkynyl, $C_{3-8}$ cycloalkyl, $C_{1-8}$ alkoxy, $C_{3-8}$ cycloalkoxy, $SO_2R_{11}$, $C(O)OR_{11}$, $C(O)R_{12}$ and $P(O)R_{13}R_{14}$, wherein alkyl and cycloalkyl are each optionally further substituted by one or more groups selected from the group consisting of halogen, hydroxy, $C_{1-8}$ alkyl, $C_{1-8}$ alkoxy, halo$C_{1-8}$ alkoxy, $C_{3-8}$ cycloalkyl and $C_{3-8}$ cycloalkoxy;
$R_{10}$ is selected from the group consisting of hydrogen, $C_{1-8}$ alkyl, $C_{3-8}$ cycloalkyl, halo $C_{1-8}$ alkyl, and $C_{1-8}$ alkoxy;
$R_{11}$ is selected from the group consisting of hydrogen, $C_{1-8}$ alkyl, $C_{3-8}$ cycloalkyl, halo $C_{1-8}$ alkyl, phenyl and p-methylphenyl;
$R_{12}$, $R_{13}$ and $R_{14}$ are each independently selected from the group consisting of hydroxy, $C_{1-8}$ alkyl, $C_{3-8}$ cycloalkyl, halo$C_{1-8}$ alkyl, $C_{1-8}$ alkoxy, amino, and $C_{1-8}$ alkyl disubstituted amino;
n is 0 or 1.

In a preferred embodiment, in the cholic acid derivatives, the stereoisomer or the pharmaceutically acceptable

salt thereof according to the present invention, the $C_{1-8}$ alkyl is preferred selected from $C_{1-6}$ alkyl, and more preferred selected from $C_{1-3}$ alkyl; the $C_{1-8}$ alkoxy is preferred selected from $C_{1-6}$ alkoxy, and more preferred selected from $C_{1-3}$ alkoxy; the $C_{3-8}$ cycloalkyl preferred selected from $C_{3-6}$ cycloalkyl; the $C_{3-8}$ cycloalkoxy is preferred selected from $C_{3-6}$ cycloalkoxy; the $C_{2-8}$ alkenyl is preferred selected from $C_{2-4}$ alkenyl; the $C_{2-8}$ alkynyl is preferred selected from $C_{2-4}$ alkynyl.

In a preferred embodiment, in the cholic acid derivatives, the stereoisomer or the pharmaceutically acceptable salt thereof according to the present invention, $R_1$ is selected from the group consisting of hydrogen, fluorine, methyl, trifluoromethyl, ethyl and isopropyl; $R_2$, $R_3$, $R_4$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$, Z and n are as defined in the compound of formula (I).

In a preferred embodiment, in the cholic acid derivatives, the stereoisomer or the pharmaceutically acceptable salt thereof according to the present invention, $R_2$ is selected from the group consisting of hydrogen, methyl, ethyl and isopropyl; $R_1$, $R_3$, $R_4$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$, Z and n are as defined in the compound of formula (I).

In a preferred embodiment, in the cholic acid derivatives, the stereoisomer or the pharmaceutically acceptable salt thereof according to the present invention, $R_3$ is selected from the group consisting of hydrogen, methyl, ethyl and isopropyl; $R_1$, $R_2$, $R_4$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$, Z and n are as defined in the compound of formula (I).

In a more preferred embodiment, in the cholic acid derivatives, the stereoisomer or the pharmaceutically acceptable salt thereof according to the present invention, $R_3$ is selected from the group consisting of hydrogen and methyl; $R_1$, $R_2$, $R_4$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$, Z and n are as defined in the compound of formula (I).

In the most preferred embodiment, the cholic acid derivatives, the stereoisomer or the pharmaceutically acceptable salt thereof according to the present invention is a compound of formula (II):

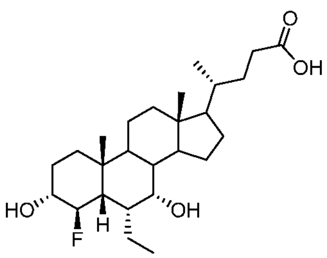

(II) .

In a more preferred embodiment, in the cholic acid derivatives, the stereoisomer or the pharmaceutically acceptable salt thereof according to the present invention, $R_1$ is hydrogen; $R_3$ is selected from the group consisting of hydrogen and methyl; $R_2$, $R_4$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$, Z and n are as defined in the compound of formula (I).

In the most preferred embodiment, the cholic acid derivatives, the stereoisomer or the pharmaceutically acceptable salt thereof according to the present invention is a compound of formula (III):

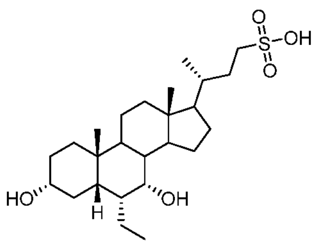

(III) .

In the most preferred embodiment, the cholic acid derivatives, the stereoisomer or the pharmaceutically acceptable salt thereof according to the present invention is a compound of formula (IV):

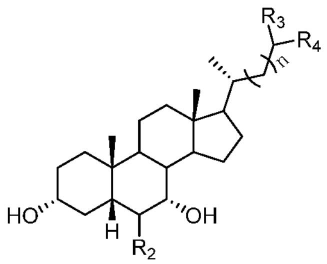

(IV)

wherein, $R_4$ is selected from the group consisting of:

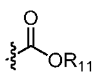 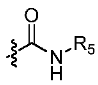 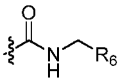 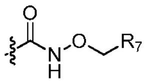 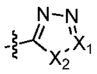 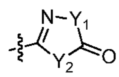 and 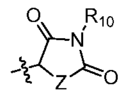 ;

$R_2$, $R_3$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$, Z and n are as defined in the compound of formula (I).

In a more preferred embodiment, the cholic acid derivatives, the stereoisomer or the pharmaceutically acceptable salt thereof according to the present invention, when $R_4$ is selected from the group consisting of

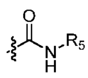 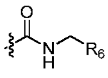 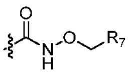 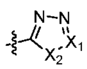 and 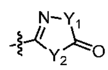 ,

n is 1; when $R_4$ is

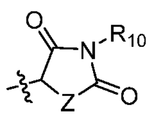 ,

n is 0;

$R_2$, $R_3$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$ and Z are as defined in the compound of formula (I).

In the most preferred embodiment, the cholic acid derivatives, the stereoisomer or the pharmaceutically acceptable salt thereof according to the present invention is selected from the group consisting of:

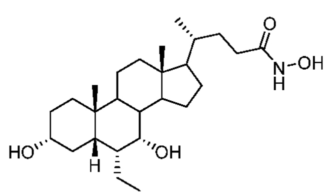 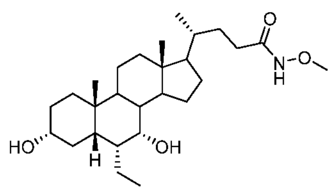 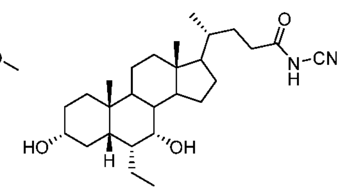

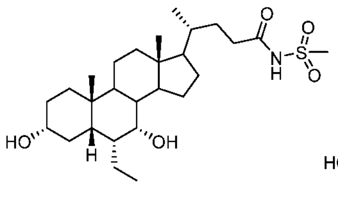 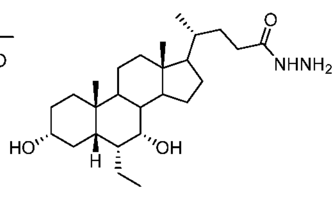 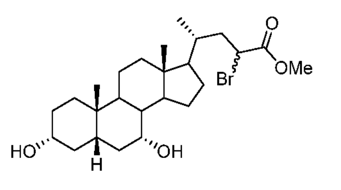

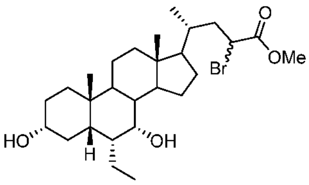

or selected from the group consisting of:

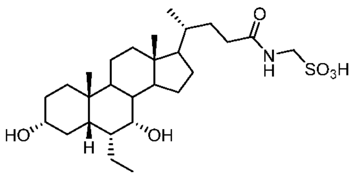

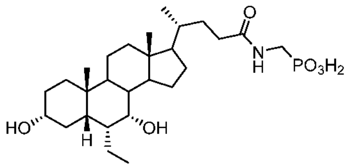

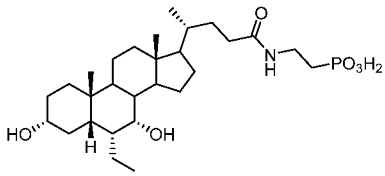

.

or selected from the compound of:

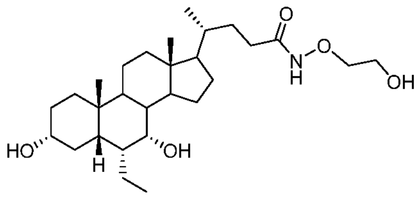

.

In a more preferred embodiment, the cholic acid derivatives, the stereoisomer or the pharmaceutically acceptable salt thereof according to the present invention is a compound of formula (V):

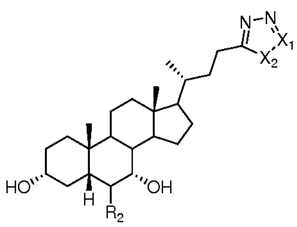

(V)

wherein, $X_1$, $X_2$, $R_2$, $R_8$, $R_9$ and $R_{10}$ are as defined in the compound of formula (I).
In the most preferred embodiment, the cholic acid derivatives, the stereoisomer or the pharmaceutically acceptable salt thereof according to the present invention is selected from the group consisting of:

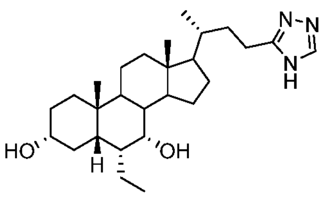

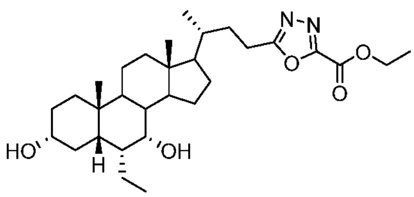

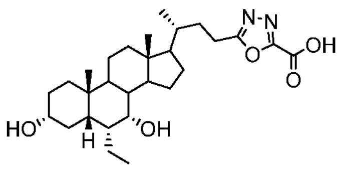 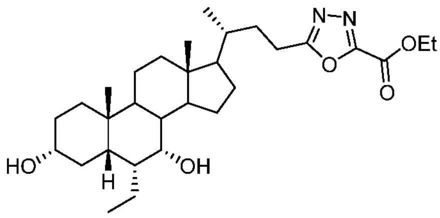 .

In a more preferred embodiment, the cholic acid derivatives, the stereoisomer or the pharmaceutically acceptable salt thereof according to the present invention is a compound of formula (VI):

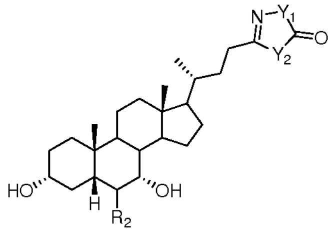

(VI)

wherein, $Y_1$, $Y_2$, $R_2$, $R_8$, $R_9$, $R_{10}$, $R_{11}$, $R_{12}$, $R_{13}$ and $R_{14}$ are as defined in the compound of formula (I).

In the most preferred embodiment, the cholic acid derivatives, the stereoisomer or the pharmaceutically acceptable salt thereof according to the present invention is selected from the group consisting of:

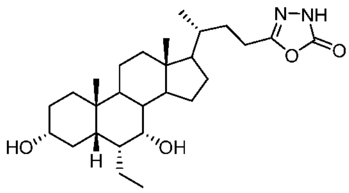 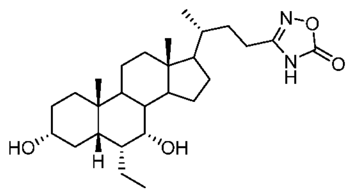 .

In a more preferred embodiment, the cholic acid derivatives, the stereoisomer or the pharmaceutically acceptable salt thereof according to the present invention is a compound of formula (VII):

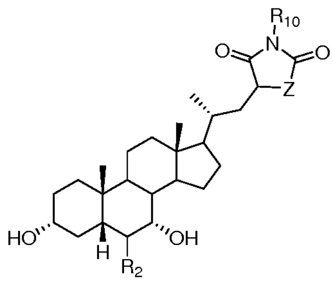

(VII)

or a compound as defined below:

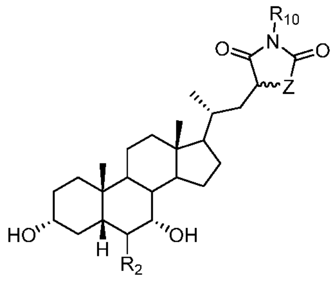

(VII)

including two isomerides as defined below:

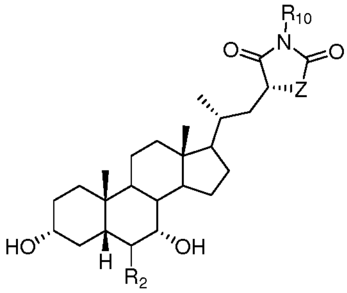

and

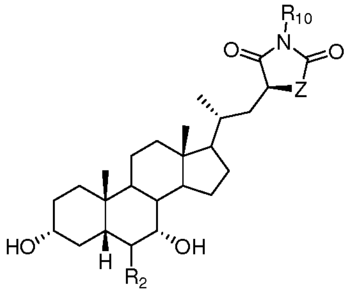

;

wherein, $R_2$, $R_8$, $R_9$, $R_{10}$, $R_{11}$, $R_{12}$, $R_{13}$, $R_{14}$ and Z are as defined in the compound of formula (I).

In the most preferred embodiment, the cholic acid derivatives, the stereoisomer or the pharmaceutically acceptable salt thereof according to the present invention is selected from the group consisting of:

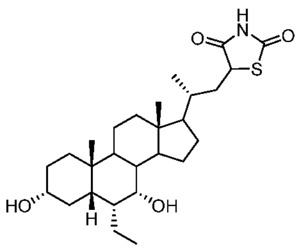

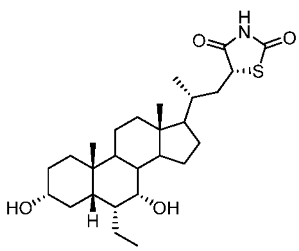

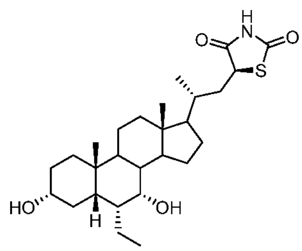

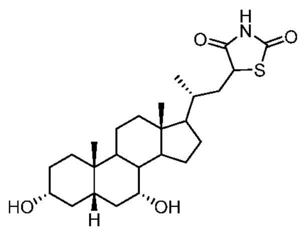

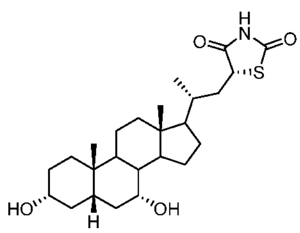

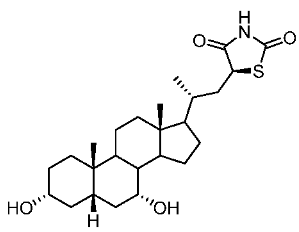

.

In another aspect, the present invention provides the the cholic acid derivatives of formula (VIII), the stereoisomer or the pharmaceutically acceptable salt thereof, the compound of formula (VIII) is as defined below:

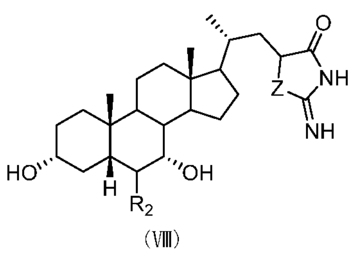

(VIII)

Z is selected from the group consisting of oxygen and sulfur atom;
$R_2$ is selected from the group consisting of hydrogen, fluorine, bromine, trifluoromethyl and $C_{1-8}$ alkyl.

In a more preferred embodiment, the cholic acid derivatives, the stereoisomer or the pharmaceutically acceptable salt thereof according to the present invention is selected from the group consisting of:

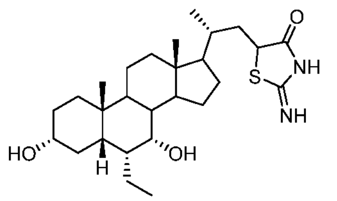
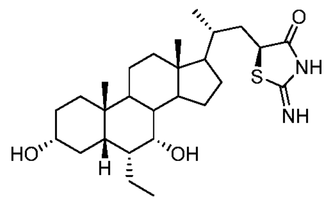
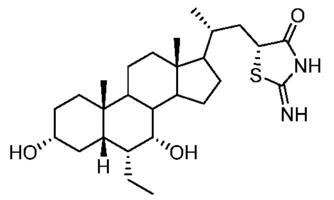
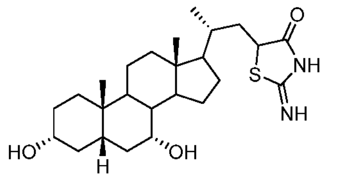
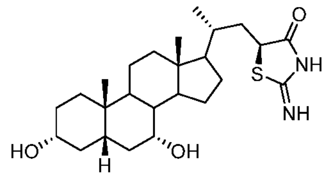
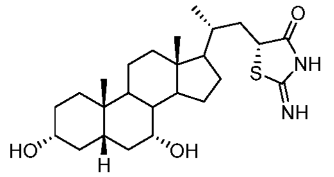

In another aspect, the present invention provides a process for preparing the aforesaid cholic acid derivatives, the stereoisomer or the pharmaceutically acceptable salt thereof, comprising the steps of:

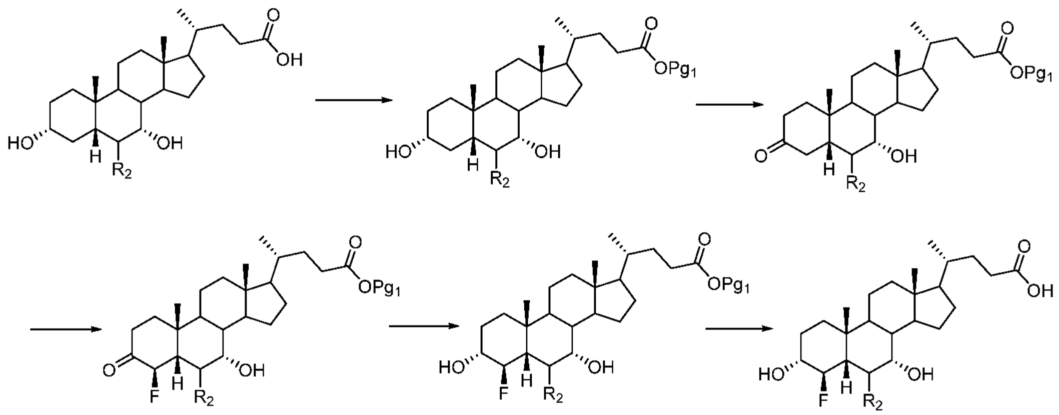

or

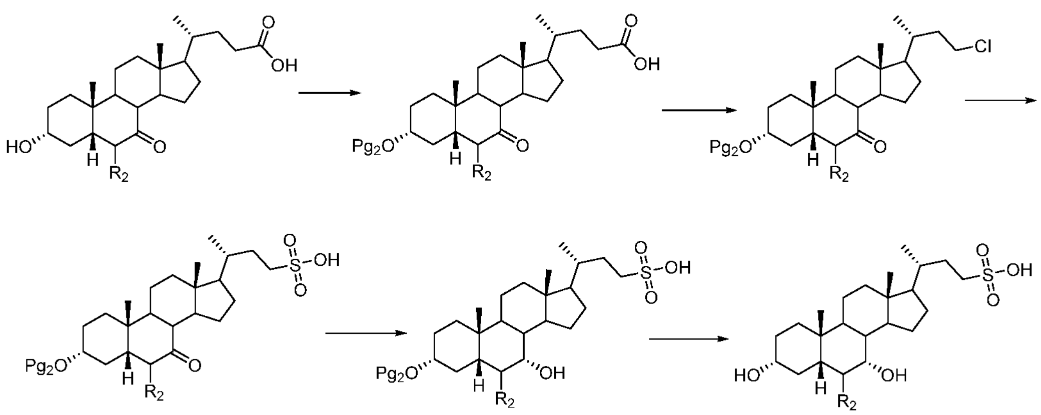

or

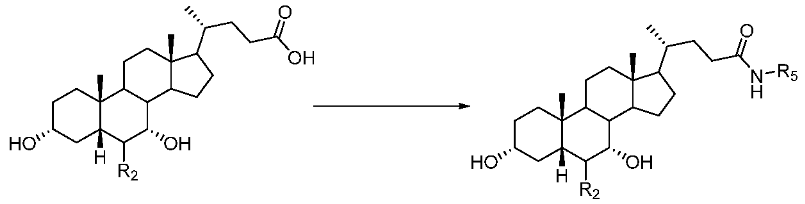

or

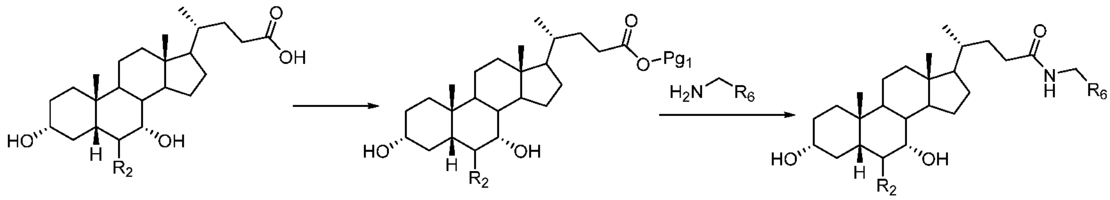

or

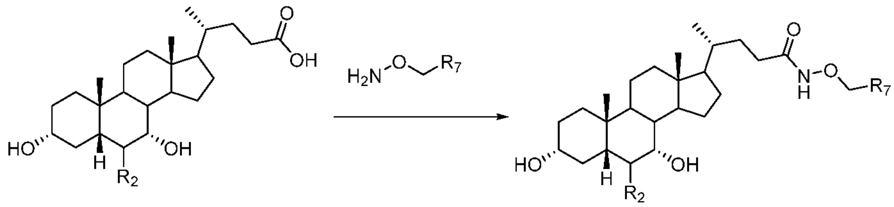

or

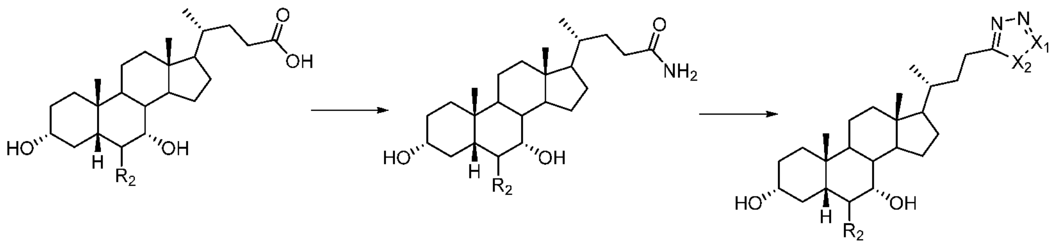

or

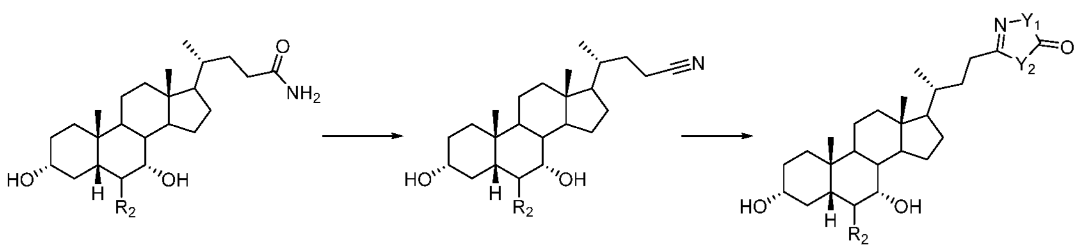

or

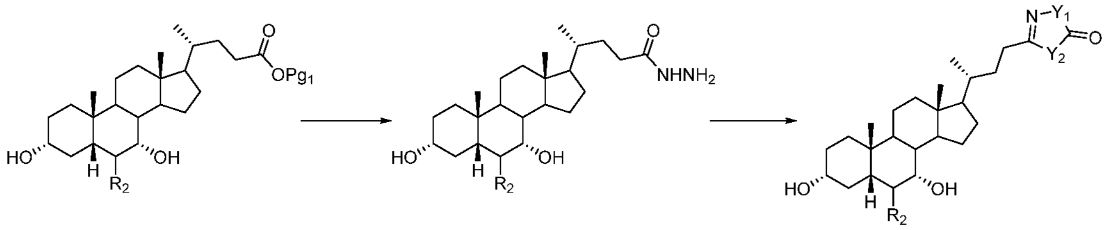

or

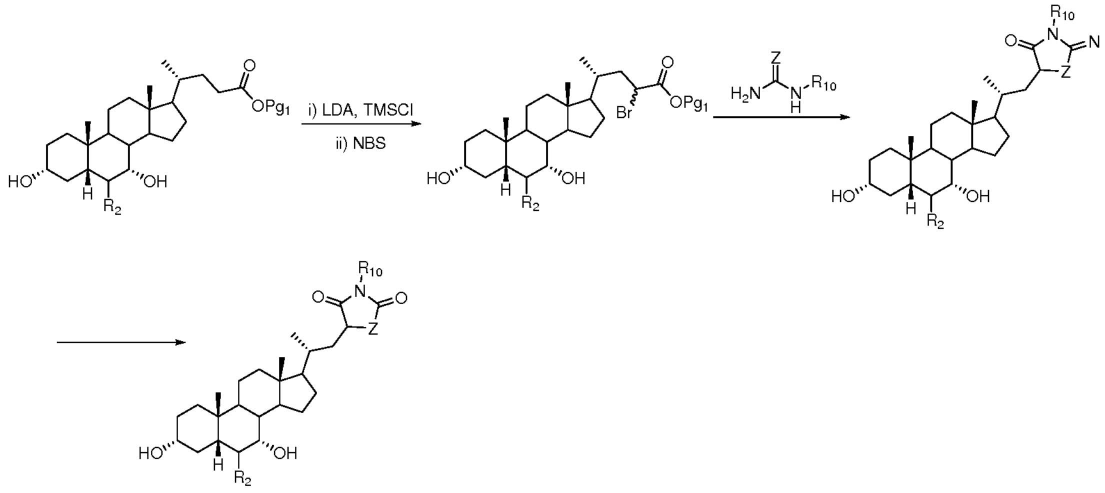


wherein, $R_2$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$ and Z are as defined in the compound of formula (I).

$Pg_1$ is hydroxyl protective group, and is preferred selected from the group consisting of $C_{1-4}$ alkyl and benzyl, $Pg_2$ is hydroxyl protective group, and is preferred selected from the group consisting of $C_{1-4}$ alkyl and acetyl.

In another aspect, the present invention provides a pharmaceutical composition comprising a therapeutically effective amount of the aforesaid cholic acid derivatives, the stereoisomer or the pharmaceutically acceptable salt thereof and a pharmaceutical acceptable carrier.

In another aspect, the present invention provides a use of the aforesaid cholic acid derivatives, the stereoisomer or the pharmaceutically acceptable salt thereof, or the aforesaid pharmaceutical composition in the preparation of a medicament for prevention or treating FXR mediated diseases and conditions.

In a preferred embodiment, FXR mediated diseases and conditions are selected from the group consisting of cardiovascular disease, hypercholesterolemia, hyperlipidemia and chronic hepatitis disease, chronic liver disease, gastrointestinal disease, nephrosis, cerebrovascular disease, metabolic disease and cancer.

In a more preferred embodiment, the chronic liver disease is selected from the group consisting of primary cirrhosis (PBC), cerebrotendinous Xanthomatosis (CTX), primary sclerosing cholecystitis (PSC), cholestasis caused by drug, intrahepatic cholestasis of pregnancy, parenteral nutrition associated cholestasis (PNAC), cholestasis of bacterial overgrowth or pyemia, autoimmune hepatitis, chronic viral hepatitis, alcoholic liver disease, non-alcoholic fatty liver disease (NAFLD), non-alcoholic steatohepatitis (NASH), graft versus host disease related to liver transplantation, regeneration of living donor liver transplantation, congenital hepatic fibrosis, choledocholithiasis, granulation liver disease, intrahepatic and extrahepatic malignant tumor, Sjogren syndrome, sarcoidosis, Wilson's disease, Gaucher's disease, hemochromatosis and $\alpha$1-antitrypsin deficiency.

In a more preferred embodiment, the gastrointestinal disease is selected from the group consisting of inflammatory bowel disease (IBD) (incluing Crohn's disease and ulcerative enteritis), irritable bowel syndrome (IBS), bacterial overgrowth, nutrient malabsorption, colonitis after reflection and microscopic colitis.

In a more preferred embodiment, the nephrosis is selected from the group consisting of diabetic nephropathy, focal segmental glomerulosclerosis (FSGS), hypertensive nephropathy, chronic glomerulitis, chronic transplant glomerulopathy, chronic interstitial nephritis and polycystic kidney disease.

In a more preferred embodiment, the cardiovascular disease is selected from the group consisting of arteriosclerosis, arteriosclerosis, atherosclerosis, dyslipidemia, hypercholesterolemia and hypertriglyceridemia.

In a more preferred embodiment, the metabolic disease is selected from the group consisting of insulin resistance, type I diabetes, type II diabetes and obesity.

In a more preferred embodiment, the cerebrovascular disease is stroke.

In a more preferred embodiment, the cancer is selected from the group consisting of colorectal cancer and liver cancer.

In another aspect, the present invention provides a method for preventing and treating FXR mediated diseases and conditions, comprising administationg of a therapeutically effective amount of the aforesaid cholic acid derivatives, the stereoisomer or the pharmaceutically acceptable salt thereof, or the aforesaid pharmaceutical composition.

## DETAILED DESCRIPTION OF THE INVENTION

Detailed description: unless otherwise stated, the following terms which are used in the description and the claims have the following meanings.

"$C_{1-8}$ alkyl" refers to a straight chain or branched chain alkyl group having 1 to 8 carbon atoms. "Alkyl" refers to a saturated aliphatic hydrocarbon group, such as methyl, ethyl, n-propyl, isopropyl, n-butyl, isobutyl, t-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl and various branched chain isomers thereof and the like.

"Cycloalkyl" refers to a saturated or partially unsaturated monocyclic or polycyclic hydrocarbon substituent. "$C_{3-8}$ cycloalkyl" refers to a cycloalkyl group having 3 to 8 carbon atoms, for example:

The non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like.

Polycyclic cycloalkyl includes a cycloalkyl having a spiro ring, fused ring and bridged ring. "Spiro cycloalkyl" refers to a polycyclic group with rings connected through one common carbon atom (called a spiro atom), wherein these rings can contain one or more double bonds, but none of the rings has a completely conjugated π electronic system. The spirocycloalkyl can be divided into mono-spiro cycloalkyl, di-spiro cycloalkyl or poly-spiro cycloalkyl according to the number of the spiro atoms shared between the rings. The non-limiting examples of the spiro cycloalkyl include:

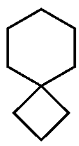 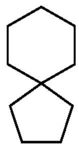 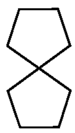 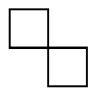 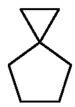 .

"Fused cycloalkyl" refers to an all-carbon polycyclic group in which each ring in the system shares an adjacent pair of carbon atoms with another ring, wherein one or more rings can contain one or more double bonds, but none of the rings has a completely conjugated π electronic system. According to the number of membered rings, the fused-cycloalkyl can be divided into bicyclic, tricyclic, tetracyclic and polycyclic fused cycloalkyl. The non-limiting examples of the fused cycloalkyl include:

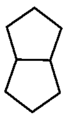 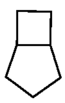 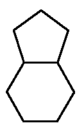 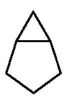 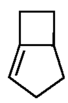 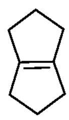 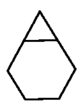 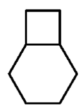 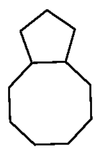 .

"Bridged cycloalkyl" refers to an all-carbon polycyclic group in which any two rings in the system share two disconnected carbon atoms, wherein these rings can contain one or more double bonds, but none of the rings has a completely conjugated π electronic system. According to the number of membered rings, the bridged cycloalkyl can be divided into bicyclic, tricyclic, tetracyclic and polycyclic bridged cycloalkyl. The non-limiting examples of the bridged cycloalkyl include:

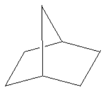 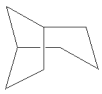 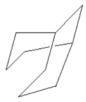 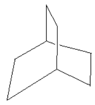 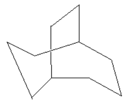 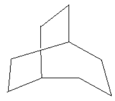 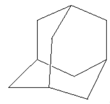 .

The cycloalkyl can be fused to the ring of aryl, heteroaryl or heterocyclyl, wherein the ring connected with the parent structure is the cycloalkyl, and the non-limiting examples include indanyl, tetrahydronaphthyl, benzocycloheptylalkyl and the like.

"Alkoxy" refers to an -O-(alkyl), wherein the alkyl is as defined above. "$C_{1-8}$ alkoxy" refers to an alkoxy having 1 to 8 carbons, and the non-limiting examples include methoxy, ethoxy, propoxy, butoxy and the like.

"Cycloalkoxy" refers to an -O-(unsubstituted cycloalkyl), wherein the cycloalkyl is as defined above. "$C_{3-8}$ cycloalkoxy" refers to a cycloalkoxy group having 3 to 8 carbons, and the non-limiting examples include cyclopropoxy, cyclobutyloxy, cyclopentyloxy, cyclohexyloxy and the like.

"Halo$C_{1-8}$ alkyl" refers to a $C_{1-8}$ alkyl group wherein hydrogens in the alkyl are substituted by fluorine, chlorine, bromine and iodine atoms, for example, difluoromethyl, dichloromethyl, dibromomethyl, trifluoromethyl, trichloromethyl, tribromomethyl and the like.

"$C(O)R_{12}$" refers to a carbonyl group substituted with $R_{12}$.

"$P(O)R_{12}R_{13}$" refers to a phosphoryl substituted with $R_{12}$ and $R_{13}$, wherein $R_{12}$ and $R_{13}$ are optionally identical or different.

"PE" refers to petroleum ether.

"EA" refers to ethyl acetate.

"THF" refers to tetrahydrofuran.

"DCM" refers to dichloromethane.

"CDI" refers to N,N-carbonyldiimidazole

"DMF" refers to N,N-dimethylformamide.

"Dioxane" refers to 1, 4-dioxane.

"DMAP" refers to 4dimethylaminopyridine.

"DIPEA" refers to diisopropylethylamine.

"TMSCl" refers to trimethylchlorosilane.

"LDA" refers to lithium diisopropylamide.

"Selectflour" refers to selective fluoride reagent.

"EDC·HCl" refers to 1-[3-(dimethylamino)propyl]-3-ethylcarbodiimide hydrochloride.

"TBTU" refers to O-benzotriazol-N,N,N',N'-tetramethyluronium tetrafluoroborate.

"NBS" refers to N-bromosuccinimide.

"Optional" or "optionally" means that the subsequently described event or the circumstance can, but need not occur. Its meaning includes the instances in which the event or the circumstance does or does not occur. For example, "heterocyclyl optionally substituted by alkyl" means that the alkyl group can be, but need not be present. Its meaning includes the instances in which heterocyclyl is unsubstituted or substituted with alkyl.

"Substituted" means that one or more hydrogen atoms in a group are each independently substituted by the corresponding number of the substituents. Apparently, the substituents are only positioned at their possible chemical positions, and the possible or impossible substitutions can be determined (through experiments or theory) by those skilled in the art without paying excessive efforts. For example, the combination of amino or hydroxy having free hydrogen and carbon atoms having unsaturated bonds (such as olefinic) may be unstable.

"Pharmaceutical composition" refers to a mixture comprising one or more of the compounds described herein or the physiological/pharmaceutical salts or prodrugs thereof and other chemical components, such as physiological/pharmaceutical carriers and excipients. The purpose of the pharmaceutical composition is to facilitate administration of a compound to an organism, which will help absorption of the active ingredient, thereby realizing biological activity.

The following examples serve to illustrate the present invention in detail and completely, but these examples should not be considered as limiting the scope of the present invention, and the present invention is not limited to the examples.

The structures of compounds in the present invention are identified by nuclear magnetic resonance (NMR) and/or liquid chromatography-mass spectrometry (LC-MS). The chemical shift of NMR is given in $10^{-6}$(ppm). NMR is determined by a Bruker AVANCE-400 instrument, and the solvents for determination are deuterated dimethylsulfoxide (DMSO-*d6*), deuterated methanol ($CD_3OD$) and deuterated chloroform ($CDCl_3$) with tetramethylsilane (TMS) as internal standard.

Liquid chromatography-mass spectrometry (LC-MS) is determined on an Agilent 1200 Infinity Series mass spectrometer. HPLC is determined on an Agilent 1200DAD high pressure liquid chromatographic instrument (Sunfire C18 150×4.6 mm chromatographic column) and a Waters 2695-2996 high pressure liquid chromatographic instrument

(Gimini C18 150×4.6 mm chromatographic column). For thin-layer silica gel chromatography (TLC), Yantai Huanghai HSGF254 or Qingdao GF254 silica gel plate is used. The dimension of the plates used in TLC is 0.15 mm to 0.2 mm, and the dimension of the plates used in product purification is 0.4 mm to 0.5 mm. Column chromatography generally uses Yantai Huanghai 200 to 300 mesh silica gel as carrier.

The starting materials used in the examples of the present invention are known and commercially available, or can be synthesized by adopting or according to the known method in the art.

Unless otherwise stated, all of the reactions of the present invention are carried out under continuous magnetic stirring in a dry nitrogen or argon atmosphere, and the solvent is dry.

## Example 1

Preparation of (4R)-4-((3R, 4R, 5S, 6R, 7R, 10R, 13R) -6-ethyl-4-fluoro-3,7-dihydroxy-10,13-dimethylhexadecahydro-1H-cyclopenta[a] phenanthrene-17-yl)pentanoic acid

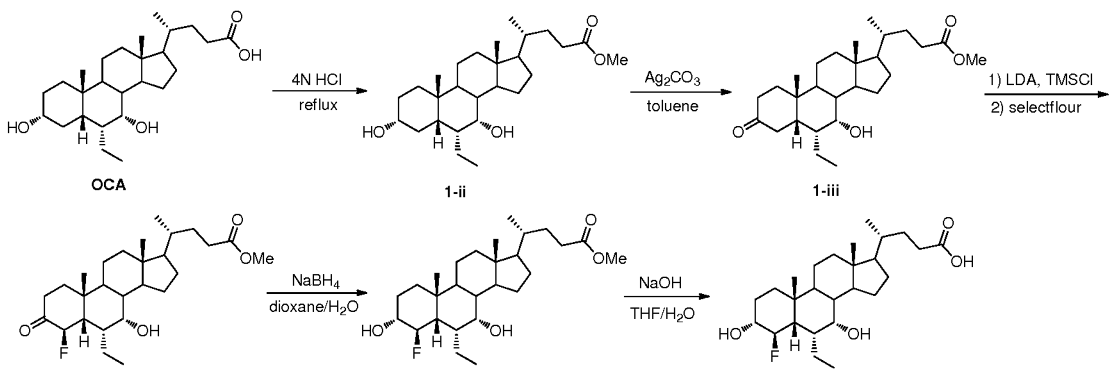


OCA 1-ii 1-iii

1-iv 1-iv 1

### Step 1:

6α-Ethyl-chenodeoxycholic acid (**OCA**, 6.0 g, 14.2 mmol) was dissolved in a solution of 4N HCl in methanol and stirred at reflux for 2 hours. After TLC (PE: EA=1: 2) showed completion of the reaction, methanol was removed by evaporation. The reaction solution was adjusted to basicity by adding saturated sodium bicarbonate, and extracted with 50 mL of ethyl acetate. The organic phase was dried over sodium sulfate, and concentrated under reduced pressure to obtain compound **1-ii** (6.0 g, 98%).

### Step 2:

Compound **1-ii** (4.0 g, 9.2 mmol) was dissolved in 40 mL of toluene, and then added with silver carbonate (5.0 g, 18.4 mmol) and celatom (10 g). The reaction solution was stirred at reflux for 24 hours. After TLC(PE: EA=1: 2) showed completion of the reaction, the reaction solution was filtered to remove celatom, and then washed with ethyl acetate several times. The organic phase was concentrated under reduced pressure and the residues were purified by column chromatography to obtain compound **1-iii** (1.0 g, 25%).

### Step 3:

Compound **1-iii** (100 mg, 0.21 mmol) was dissolved in 2 mL of tetrahydrofuran, and then added with TMSCl (200 μL, 1.05 mmol) and LDA (90 μL, 0.42 mmol) at -78 °C. The mixture was stirred for 3 hours, quenched with saturated sodium bicarbonate and extracted with ethyl acetate. The organic phase was dried over sodium sulfate and concentrated under reduced pressure to obtain an intermediate. The resulting product was dissolved in 2 mL of acetonitrile and stirred at room temperature for 1 hour after selectflour (105 mg, 0.29 mmol) was added. After TLC (PE: EA = 5: 1) showed completion of the reaction, 10 mL of water was added, and the reaction solution was extracted with ethyl acetate. The organic phase was concentrated under reduced pressure to obtain 100 mg of compound **1-iv**, and the crude product was used directly in the next step.

**Step 4:**

Compound **1-iv** (300 mg, 0.6 mmol) was dissolved in dioxane / $H_2O$ (10 mL / 0.5 mL), and sodium borohydride (49 mg, 1.3 mmol) was added in portions. The reaction mixture was stirred overnight at room temperature. One equivalent of sodium borohydride was added in the reaction mixture, and the stirring was continued for 4 hours. After TLC (PE: EA = 5: 1) showed the disappearance of the starting material, the reaction was quenched with 10 mL of water, extracted with ethyl acetate. The organic phase was dried over sodium sulfate and purified by column chromatography to obtain compound **1-v** (40 mg, 15%).

**Step 5:**

Compound **1-v** (40 mg, 0.088 mmol) was dissolved in 1 mL of tetrahydrofuran, and added with 1 mL of 1 N NaOH . The reaction solution was stirred at room temperature for 2 hours. After TLC (PE: EA = 1: 1) showed completion of the reaction, the reaction solution was adjusted to acidity by adding 1N HCl, and extracted with ethyl acetate. The organic phase was dried over sodium sulfate and purified by column chromatography to obtain compound 1 (5 mg, 12%).
$^1$H NMR (400 MHz, $CDCl_3$) $\delta$ 5.35 - 5.13 (m, 1H), 3.75 (s, 1H), 3.56 - 3.36 (m, 1H), 2.40 - 2.28 (m, 1H), 2.23 - 2.11 (m, 1H), 0.90 (s, 3H), 0.88 - 0.83 (m, 6H), 0.59 (s,3H).
$^{13}$C NMR (101 MHz, $CDCl_3$) $\delta$ 178.50, 99.65 (d, *J*= 168.8 Hz), 74.89 (d, *J*= 20.0 Hz), 70.96, 55.78, 50.45, 49.37 (d, *J*= 13.6 Hz), 42.77, 42.10, 40.33, 39.48, 39.00 (d, *J*= 9.4 Hz), 35.39, 35.33, 34.44, 30.78, 29.69, 28.15, 26.02 (d, *J*= 8.5 Hz), 24.28 (d, *J*= 8.3 Hz), 23.60, 23.28, 20.75, 18.23, 12.48, 11.83.
$^{19}$F NMR (376 MHz, $CDCl_3$) $\delta$ -188.73.

**Example 2**

Preparation of (4R)-4-((3R,5S,6R,7R,10S,13R)-6-ethyl-3,7-dihydroxy-10,13-dimethylhexadecahydro-1 H-cyclopenta[a]phenanthrene-17-yl) -N-hydroxypentanamide

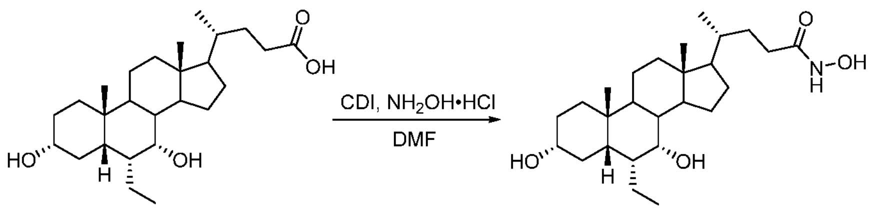


**OCA** **2**

6$\alpha$-Ethyl- chenodeoxycholic acid (**OCA**, 100 mg, 0.24 mmol) was dissolved in 2 ml of N, N-dimethylformamide and added with N, N-carbonyldiimidazole (77 mg, 0.48 mmol). The reaction mixture was stirred at room temperature for 0.5 hour. Then hydroxylamine hydrochloride (66 mg, 0.95 mmol) was added, and the reaction was continued at room temperature for 0.5 hour until completed. The reaction mixture was quenched with water, and extracted with ethyl acetate three times. The organic phase was dried over anhydrous sodium sulfate and filtered. The filtrate was concentrated under reduced pressure and the residues were purified by column chromatography to obtain compound compound **2** (16 mg, 15%).
$^1$H NMR (400 MHz, DMSO) $\delta$ 4.29 (d, *J*= 4.2 Hz, 1H), 4.04 (d, *J*= 5.0 Hz, 1H), 3.50 (brs, 1H), 3.20 -3.10 (m, 2H), 2.05 - 1.61 (m), 1.60 - 0.76 (m), 0.60 (s, 3H).
$^{13}$C NMR (101 MHz, DMSO) $\delta$ 169.99, 71.08, 68.89, 56.02, 50.60, 45.83, 42.50, 41.77, 36.01, 35.70, 35.42, 34.05, 33.14, 31.94, 30.93, 29.68, 29.44, 28.29, 23.57, 22.63, 20.89, 18.76, 12.22, 12.17.

**Example 3**

Preparation of (4R)-N-cyano-4-((3R, 5S, 6R, 7R, 10S, 13R)-6-ethyl-3,7-dihydroxy-10,13-dimethylhexadecahydro-1H-cyclopenta[a] phenanthrene-17-yl)pentanamide

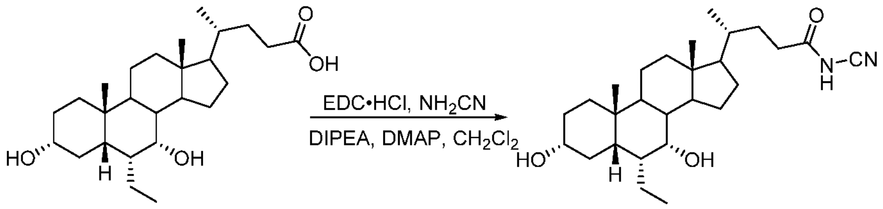


**OCA** **3**

6α-Ethyl- chenodeoxycholic acid (**OCA**, 60 mg, 0.14mmol), DMAP (21 mg, 0.17 mmol), EDC·HCl (38 mg, 0.20 mmol) and $NH_2CN$ (12 mg, 0.28 mmol) were dissolved in 2 ml of dichloromethane, and added with DIPEA (50 μL, 0.28 mmol). The reaction mixture was stirred at room temperature overnight until completed. The reaction solution was diluted with dichloromethane, washed with dilute hydrochloric acid and saturated brine successively. The organic phase was dried over anhydrous sodium sulfate and filtered. The filtrate was concentrated under reduced pressure and the residues were purified by column chromatography to obtain compound **3** (25 mg, 39%).
$^1H$ NMR (400 MHz, DMSO) δ 4.28 (brs, 1H), 3.50 (brs, 1H), 3.13 (brs, 1H), 2.30 (dd, *J*= 9.6, 5.1 Hz, 2H), 2.20 (dd, *J*= 14.1, 5.8 Hz, 2H), 1.96 - 1.59 (m), 1.60 - 0.76 (m), 0.61 (s, 3H).
$^{13}C$ NMR (101 MHz, DMSO) δ 175.28, 71.08, 68.89, 55.92, 50.59, 45.82, 42.53, 41.76, 36.02, 35.70, 35.30, 34.04, 33.14, 32.40, 30.92, 29.44, 28.24, 23.57, 22.63, 20.89, 18.69, 12.18, 12.17.

**Example 4**

Preparation of (4R)-4-((3R,5S,6R,7R,10S,13R)-6-ethyl-3,7-dihydroxy-10,13-dimethylhexadecahydro-1 H-cyclopenta[a]phenanthrene-17-yl) -N-methoxypentanamide

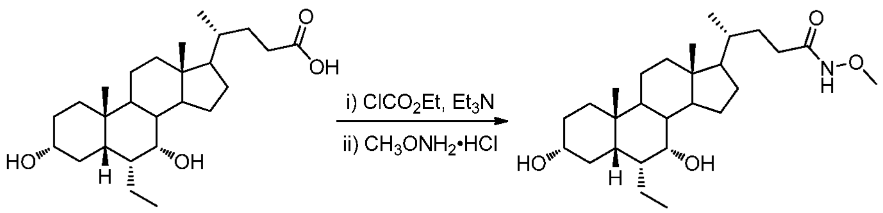


**OCA** **4**

6α-Ethyl- chenodeoxycholic acid (**OCA**, 42 mg, 0.1 mmol) was dissolved in 1 mL of N,N-dimethylformamide, and added with triethylamine (0.03 mL, 0.2 mmol) . Then ethyl chloroformate (0.01 mL, 0.11 mmol) was added in an ice bath and stirred for 0.5 h. Methoxylamine hydrochloride (9 mg, 0.11 mmol) was dissolved in 1 mL of N, N-dimethylformamide, and then added with triethylamine (0.03 mL, 0.2 mmol). The solution was added dropwise to the reaction mixture above and the reaction temperature was gradually increased to room temperature. The reaction was completed after 3-4 h. The reaction solution was washed with saturated brine, and extracted with ethyl acetate three times. The organic phases were combined and dried over anhydrous sodium sulfate, filtered and the filtrate was concentrated by column chromatography to obtain compound **4** (19.2 mg, 43%).
$^1H$ NMR (400 MHz, $CDCl_3$) δ 3.69 (brs, 3H), 3.63 (brs, 1H), 3.40 - 3.27 (m, 1H), 1.96 -1.02 (m), 1.00 - 0.75 (m), 0.59 (s, 3H).
$^{13}C$ NMR (101 MHz, MeOD) δ 171.86, 71.81, 69.79, 62.89, 55.87, 50.29, 45.57, 42.38, 41.78, 40.19, 39.66, 35.41, 35.26, 33.15, 33.05, 31.50, 29.88, 29.39, 27.89, 23.18, 22.39, 22.11, 20.60, 17.51, 10.87, 10.66.

**Example 5**

Preparation of ((4R)-4-((3R,5S,6R,7R,10S,13R)-6-ethyl-3,7-dihydroxy-10,13-dimethylhexadecahydro-1 H-cyclopenta[a]phenanthrene-17-yl)pentanoylamino) methanesulfonic acid

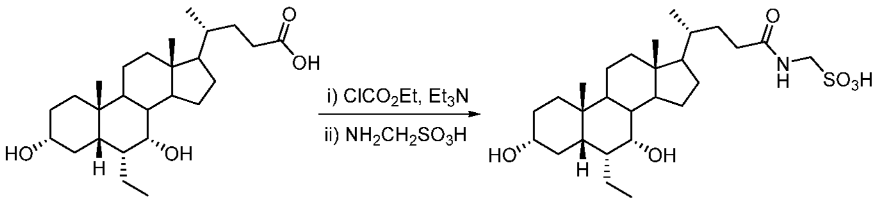


OCA 5

6α-Ethyl- chenodeoxycholic acid (**OCA**, 42 mg, 0.10 mmol) was dissolved in 1 mL of dry N, N-dimethylformamide, and added with triethylamine (28 μL, 0.20 mmol) and ethyl chloroformate (11 μL, 0.11 mmol) successively in an ice-water bath. The reaction mixture was stirred at this temperature for 30 minutes. After the addition of aminomethanesulfonic acid (12 mg, 0.11 mmol), the reaction mixture was increased to room temperature and stirred for 5 hours. After completion of the reaction, the reaction solution was concentrated under reduced pressure, and the crude product was purified by column chromatography to obtain compound **5** (35 mg, 68%).
$^1H$ NMR (400 MHz, MeOD) δ 4.42 - 4.25 (m, 2H), 3.67 (brs, 1H), 3.33 (brs, 1H), 2.48 - 2.31 (m, 1H), 2.28 - 2.13 (m, 1H), 2.11 - 1.70 (m), 1.68 - 0.84 (m), 0.71 (s, 3H).
$^{13}C$ NMR (101 MHz, MeOD) δ 175.42, 71.84, 69.86, 56.00, 55.50, 50.30, 45.57, 42.41, 41.75, 40.19, 39.69, 35.59, 35.45, 35.30, 33.15, 33.07, 32.71, 31.59, 29.93, 27.95, 23.25, 22.51, 22.12, 20.64, 17.68, 11.03, 10.77.

**Example 6**

Preparation of (((4R)-4-((3R,5S,6R,7R,10S,13R)-6-ethyl-3,7-dihydroxy-10,13-dimethylhexadecahydro-1 H-cyclopenta[a]phenanthrene-17-yl)pentanoylamino) methyl)phosphonic acid

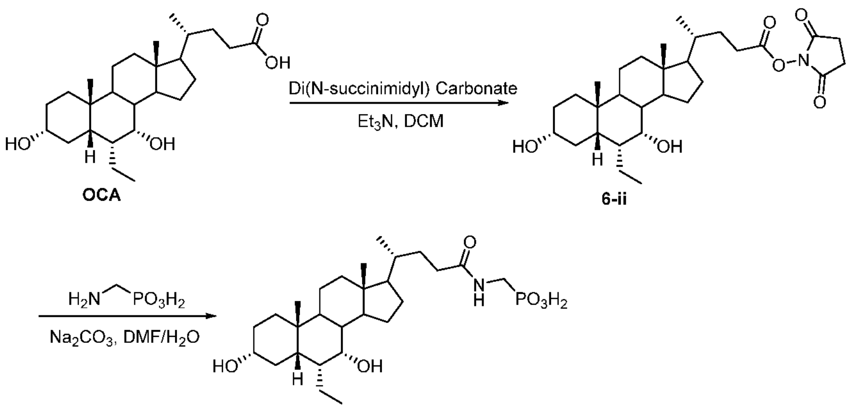


OCA 6-ii

6

**Step 1:**

6α-Ethyl-chenodeoxycholic acid (**OCA**, 40 mg, 0.046 mmoL) was dissolved in dichloromethane, and added with di(N-succinimidyl) carbonate (20 mg, 0.051 mmol) and triethylamine (40 μL, 0.14 mmol) successively. The reaction mixture was stirred at room temperature for 4 hours. After TLC (PE: EA = 1: 2) showed the disappearance of starting material, 2 mL of water and 10 mL of dichloromethane were added. The organic phase was dried over sodium sulfate and concentrated under reduced pressure to obtain compound **6-ii.**

**Step 2:**

The compound **6-ii** was dissolved in DMF/$H_2O$ (1 mL/1 mL), and added with sodium carbonate (12 mg, 0.11 mmol) and aminomethyl phosphoric acid. The reaction mixture was stirred at room temperature for 24 hours. After TLC (PE: EA = 2: 1) showed completion of the reaction, the reaction solution was adjusted to acidity by adding 1N HCl, and 10 mL of ethyl acetate was added. The organic phase was washed with 2 mL of water three times, dried over sodium sulfate and concentrated under reduced pressure to obtain compound **6** (5.4 mg, 22%).
$^1H$ NMR (400 MHz, MeOD) δ 3.55 (s, 1H), 3.48 (d, *J*= 12.5 Hz, 2H), 3.23 - 3.19 (m, 1H), 0.88 (d, *J*= 6.4 Hz, 3H), 0.85 -

0.74 (m, 6H), 0.60 (s, 3H).
$^{13}C$ NMR (101 MHz, MeOD) δ 175.22, 71.81, 69.81, 56.01, 50.28, 45.57, 42.36, 41.77, 40.18, 39.67, 36.78, 35.52, 35.39, 35.25, 33.15, 33.04, 32.48, 31.71, 29.87, 27.89, 23.18, 22.36, 22.10, 20.59, 17.52, 10.85, 10.63.
$^{31}P$ NMR (162 MHz, MeOD) δ 20.49 (s).

**Example 7**

Preparation of (2-((4R)-4-((3R,5S,6R,7R,10S,13R)-6-ethyl-3,7-dihydroxy-10,13-dimethylhexadecahydro-1 H-cyclopenta[a]phenanthrene-17-yl)pentanoylamino)ethyl) phosphonic acid

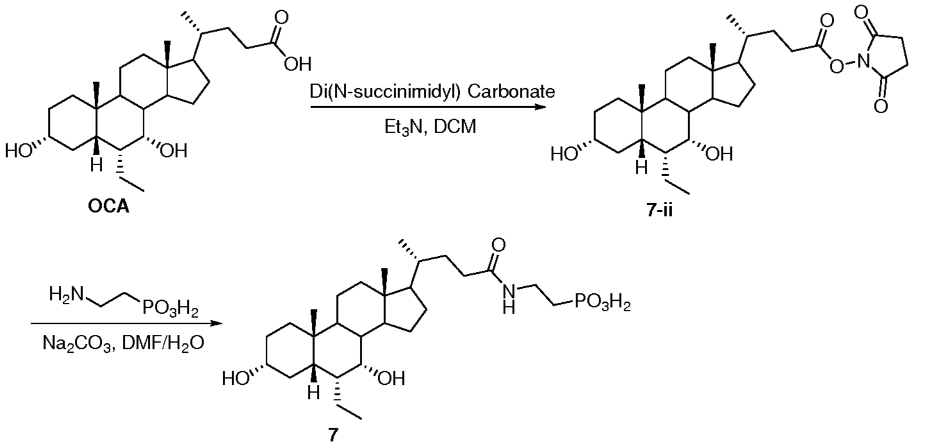


OCA, 7-ii, 7

**Step 1:**

6α-Ethyl-chenodeoxycholic acid (**OCA**, 40 mg, 0.046 mmoL) was dissolved in dichloromethane, and added with di(N-succinimidyl) carbonate (20 mg, 0.051 mmol) and triethylamine (40 μL, 0.14 mmol) successively. The reaction mixture was stirred at room temperature for 4 hours. After TLC (PE: EA = 1: 2) showed the disappearance of starting material, 2 mL of water and 10 mL of dichloromethane were added. The organic phase was dried over sodium sulfate and concentrated under reduced pressure to obtain compound **7-ii.**

**Step 2:**

The compound **7-ii** was dissolved in DMF/$H_2O$ (1 mL/1 mL), and added with sodium carbonate (12 mg, 0.11 mmol) and aminoethyl phosphoric acid. The reaction mixture was stirred at room temperature for 24 hours. After TLC (PE: EA = 2: 1) showed completion of the reaction, the reaction solution was adjusted to acidity by adding 1N HCl. 10 mL of ethyl acetate was added, and the organic phase was washed with 2 mL of water three times, dried over sodium sulfate and concentrated under reduced pressure to obtain compound **7** (5.4 mg, 22%).
$^{1}H$ NMR (400 MHz, MeOD) δ 3.62 - 3.52 (m, 1H), 3.38 - 3.27 (m, 1H), 3.24 - 3.20 (m, 2H), 0.92 - 0.84 (m, 3H), 0.84 - 0.76 (m, 6H), 0.60 (s, 3H).
$^{13}C$ NMR (101 MHz, MeOD) δ 175.31, 71.81, 69.81, 60.13, 55.96, 50.28, 45.58, 42.36, 41.78, 40.19, 39.66, 35.51, 35.39, 35.25, 33.15, 33.05, 31.79, 29.87, 29.32, 27.90, 23.17, 22.34, 22.10, 20.58, 17.52, 13.05, 10.84, 10.61.
$^{31}P$ NMR (162 MHz, MeOD) δ 25.30 (s).

**Example 8**

Preparation of (4R)-4-((3R, 5S, 6R, 7R, 10S, 13R)-6-ethyl-3,7-dihydroxy-10,13-dimethylhexadecahydro-1H-cyclopenta[a]phenant hrene-17-yl)-N-(2-hydroxyethoxy)pentanamide

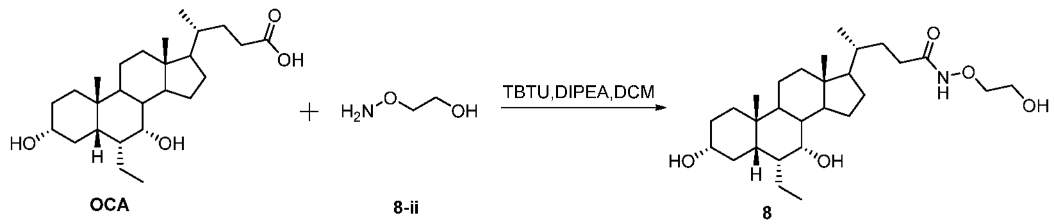


OCA 8-ii 8

6α-Ethyl-chenodeoxycholic acid (**OCA**, 30 mg, 0.07 mmol) and compound **8-ii** (6.5 mg, 0.085 mmoL) were dissolved in dichloromethane, and added with TBTU (40 mg, 0.11 mmoL) and DIPEA (30 μL, 0.21 mmoL) successively. The reaction solution was stirred at room temperature overnight. After TLC showed the disappearance of starting material, water was added, and the reaction solution was extracted with ethyl acetate. The organic phase was concentrated under reduced pressure and the residues were purified by column chromatography to obtain compound **8**(10 mg, 30%).

$^1$H NMR (400 MHz, MeOD) δ 3.95 - 3.85 (m, 1H), 3.74 (dt, *J*= 16.4, 4.7 Hz, 2H), 3.67 (s, 1H), 3.35 - 3.32 (m, 1H), 1.00 (d, *J*= 6.5 Hz, 3H), 0.95 - 0.89 (m, 6H), 0.72 (s, 3H).

$^{13}$C NMR (101 MHz, MeOD) δ 172.75, 77.45, 71.80, 69.78, 59.02, 55.86, 50.29, 48.46, 45.57, 42.37, 41.77, 40.17, 39.65, 35.40, 35.25, 33.15, 33.04, 31.49, 29.87, 29.32, 27.90, 23.17, 22.36, 22.10, 20.59, 17.47, 10.85, 10.64.

**Example 9**

Preparation of (3R)-3-((3R,5S,6R,7R,10S,13R)-6-ethyl-3,7-dihydroxy-10,13-dimethylhexadecahydr o-1H-cyclopenta[a]phenanthrene-17-yl)butane-1-sulfonic acid

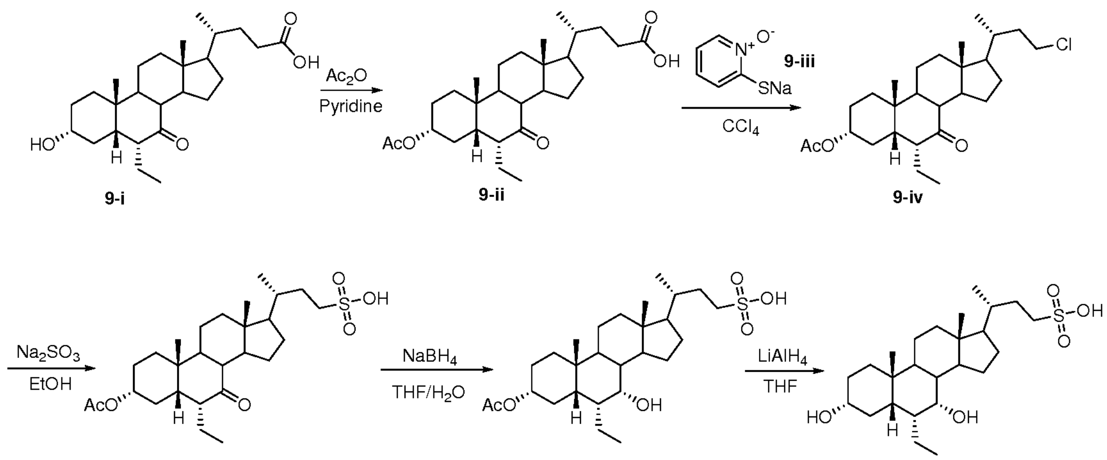


9-i 9-ii 9-iv 9-v 9-vi 9

**Step 1:**

Compound **9-i** (1.5 g, 3.58 mmol) was dissolved in 20 mL of pyridine, and added with 15 mL of acetic anhydride. The reaction solution was stirred at room temperature overnight. After TLC (PE: EA = 1: 1) showed completion of the reaction, the solvent was removed by evaporation, and the residues was purified by column chromatography to obtain compound **9-ii** (700 mg, 42%).

**Step 2:**

Compound **9-ii** (100 mg, 0.21 mmol) was dissolved in 2 mL of dichloromethane, and added with a drop of DMF, then added with oxalyl chloride (18μL, 0.21 mmoL). The reaction solution was stirred at room temperature for 1 hour before TLC (PE: EA = 5: 1) showed completion of the reaction. The solvent was removed by evaporation, then the crude product was dissolved in 2 mL of acetonitrile, and added with compound **9-iii** (38 mg, 0.25 mmol) and DMAP (1 mg, 0.05%). The mixture was stirred under nitrogen atmosphere for 2 hours. After TLC (PE: EA= 5: 1) showed completion of the reaction, compound **9-iv** (110 mg, 90%) was obtained by column chromatography directly.

**Step 3:**

Compound **9-iv** (300mg, 0.66 mmol) was dissolved in 12 mL of ethanol, and added with 12mL of sodium sulfite aqueous solution (20%). The reaction solution was heated to reflux 24 hours. After TLC (PE: EA = 10: 1) showed completion of the reaction, 20 mL of water was added, and the solution was acidity. The reaction solution was extracted with ethyl acetate, dried over sodium sulfate and concentrated under reduced pressure to obtain compound **9-v** (200 mg, 76%).

**Step 4:**

Compound **9-v** (70mg, 0.14mmol) was dissolved in THF/$H_2O$ (4mL/0.4mL) and stirred at room temperature for 2 hours after addition of 18 mg of sodium borohydride. The reaction was quenched with water, and extracted with ethyl acetate. The organic phase was concentrated under reduced pressure, and compound **9-vi** (30 mg, 50%) was obtained by column chromatography.

**Step 5:**

Compound **9-vi** (30 mg, 0.06mmol) was dissolved in THF, and added with lithium aluminum hydride (10 mg, 0.27 mmol) under nitrogen atmosphere. The resulting solution was stirred at 70 °C overnight, then quenched with water and extracted with ethyl acetate. The organic phase was concentrated under reduced pressure, and compound **9** (1 mg, 3%) was obtained by column chromatography.
$^1$H NMR (400 MHz, $CDCl_3$) δ 3.70 - 3.51 (m, 3H), 3.42 - 3.27 (m, 1H), 0.91 - 0.87 (m, 3H), 0.85 - 0.79 (m, 6H), 0.61 (s, 3H).
$^{13}$C NMR (101 MHz, $CDCl_3$) δ 72.36 70.93, 60.90, 56.38, 50.58, 45.25, 42.83, 41.23, 40.09, 39.67, 39.02, 35.56, 34.07, 33.29, 32.94, 30.70, 29.69, 28.40, 23.74, 23.15, 22.25, 20.77, 18.84, 11.77, 11.65.

**Example 10**

Preparation of 5-((3R)-3-((3R,5S,6R,7R,10S,13R)-6-ethyl-3,7-dihydroxy-10,13-dimethylhexadecane-1H-cyclopenta[a]phenanthrene-17-yl)butyl)-1,3,4-oxadiazol-2 (3H) -one

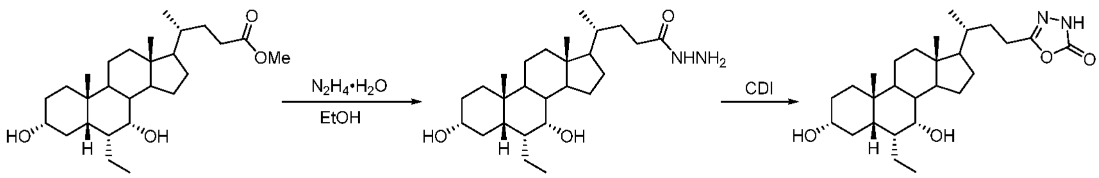

10-i 10-ii 10

**Step 1:**

Methyl 6α-ethyl-chenodeoxycholicate **10-i** (96 mg, 0.22 mmol) was dissolved in 2.5 mL of ethanol, and added with hydrazine hydrate (0.5 mL, 85%). The mixture was heated to reflux for 7 hours until completed basically. After cooling to the room temperature, the mixture was washed with saturated brine and extracted with ethyl acetate three times. The organic phases were combined and dried over anhydrous sodium sulfate, and then filtered. The filtrate was concentrated under reduced pressure to obtain compound 10-ii(87 mg, 91%).

**Step 2:**

The above crude product (43 mg, 0.10 mmol) was dissolved in 2 mL of dry tetrahydrofuran, and then added with N, N-carbonyldiimidazole (19 mg, 0.12 mmol) . The mixture was stirred at room temperature overnight. After completion of the reaction, the mixture was washed with saturated brine and extracted with dichloromethane three times. The organic phases were combined and dried over anhydrous sodium sulfate, and then filtered. The filtrate was concentrated under reduced pressure to obtain crude product which was purified by column chromatography to obtain compound **10** (20 mg, 43%).
$^1$H NMR (400 MHz, $CDCl_3$) δ 9.64 (s, 1H), 3.65 (brs, 1H), 3.44 - 3.25 (m, 1H), 2.54 (ddd, *J*= 15.2, 10.5, 4.5 Hz, 1H), 2.38 (ddd, *J*= 15.7, 9.9, 6.2 Hz, 1H), 2.12 - 1.01 (m), 1.00 - 0.75 (m), 0.59 (s, 3H).

$^{13}C$ NMR (101 MHz, $CDCl_3$) δ 158.71, 155.39, 72.31, 71.14, 55.62, 50.45, 45.17, 42.78, 41.23, 39.98, 39.60, 35.54, 35.51, 35.30, 33.91, 33.28, 31.38, 30.58, 28.22, 23.67, 23.39, 23.14, 22.23, 20.75, 18.25, 11.79, 11.65.

**Example 11**

Preparation of (3R,5S,6R,7R,10S,13R)-17-((R)-4-(4H-1,2,4-triazol-3-yl) butan-2-yl) -6-ethyl-10,13-dimethylhexadecahydro-1H-cyclopenta[a] phenanthrene-3,7-diphenol

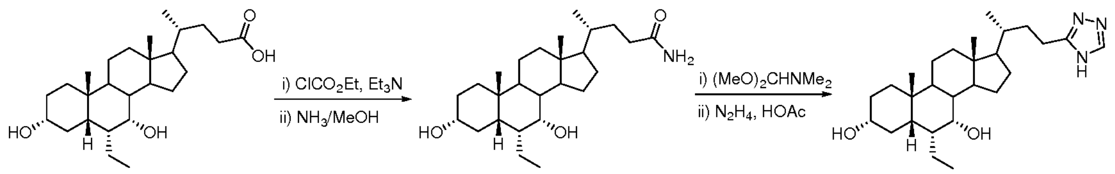


**OCA** **11-i** **11**

**Step 1:**

6α-Ethyl-chenodeoxycholic acid (**OCA**, 42 mg, 0.10 mmol) was dissolved in 1 mL of dry N, N-dimethylformamide, and added with triethylamine (28 μL, 0.20 mmol) and ethyl chloroformate (11 μL, 0.11 mmol) successively in a ice water bath. The mixture was stirred for 30 minutes, and then added with 2.0 M solution of ammonia in methanol (0.10 mL, 0.20 mmol). The reaction mixture was warmed to room temperature and stirred for 2 hours untile completed. The reaction solution was concentrated under reduced pressure to give a crude product which was used directly in the next step.

**Step 2:**

N,N-dimethylformamide dimethyl acetal (2 mL) was added to the above crude product, and the mixture was heated to reflux for 2 hours. After completion of the reaction, the mixture was cooled to room temperature and concentrated under reduced pressure. The resulting crude product was dissolved in 2 mL of acetic acid and then added with hydrazine hydrochloride (21 mg, 0.20 mmol). The reaction solution was heated to 90°C and stirred for 90 minutes. The reaction solution was cooled to room temperature, quenched with water, and extracted with ethyl acetate. The organic phase was washed with saturated aqueous $NaHCO_3$, dried over anhydrous sodium sulfate, and filtered. The filtrate was concentrated under reduced pressure and the residues were purified by column chromatography to obtain compound 11 (21 mg, 47%).
$^1H$ NMR (400 MHz, MeOD) δ 3.67 (brs, 1H), 2.94 - 2.80 (m, 1H), 2.73 - 2.66(m, 1H), 2.09 - 0.97 (m), 0.96 - 0.85 (m, 6H), 0.70 (s, 3H).
$^{13}C$ NMR (101 MHz, MeOD) δ 124.72, 71.80, 69.79, 55.87, 50.28, 45.57, 42.38, 41.77, 40.18, 39.66, 35.40, 35.25, 34.19, 33.15, 33.05, 29.88, 29.52, 27.92, 23.18, 22.37, 22.10, 20.59, 17.50, 10.83, 10.64.

**Example 12**

Preparation of 3-((3R)-3-((3R, 5S, 6R, 7R, 10S, 13R) -6-ethyl-3,7-dihydroxy-10,13-dimethylhexadecahydro-1H-cyclopenta[a] phenanthrene-17-yl) butyl)-1,2,4-oxadiazol-5(4H)-one

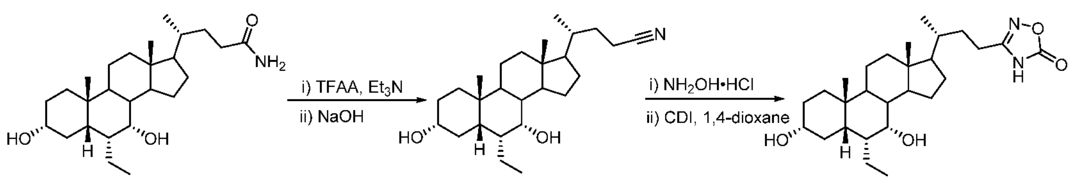


**12-i** **12-ii** **12**

**Step 1:**

6α-Ethyl-chenodeoxycholic amide **12-i**(210 mg, 0.50 mmol) was dissolved in 5 ml of tetrahydrofuran, and then added with triethylamine(0.56 mL, 4.0 mmol) and trifluoroacetic anhydride (0.28 mL, 2.0 mmol) successively in an ice-water bath. The mixture was stirred at room temperature for 3 hours. After completion of the reaction, the reaction solution was quenched with saturated aqueous $NaHCO_3$ solution and extracted with ethyl acetate twice. The organic phase was concentrated under reduced pressure and the residues were dissolved in 5 ml of tetrahydrofuran, and then 5 ml of 0.5 N aqueous solution of sodium hydroxide was added. The mixture was heated to 60 °C and stirred for 3 hours before extracted with ethyl acetate after completion. The organic phase was dried over anhydrous $Na_2SO_4$ and filtered. The filtrate was concentrated under reduced pressure and the residues were purified by column chromatography to obtain cyano compound **12-ii**(167 mg, 83%).

**Step 2:**

Compound **12-ii** (20 mg, 0.05 mmol), hydroxylamine hydrochloride (4 mg, 0.055 mmol) and $NaHCO_3$(5 mg, 0.06 mmol) were added with 2 ml of methanol. The mixture was stirred at 120 °C under microwave for 15 hours. After about half of the reaction was completed, hydroxylamine hydrochloride (4 mg, 0.055 mmol) and $NaHCO_3$(5 mg, 0.06 mmol) were added, and the reaction was continued at 120 °C under microwave for 12 hours until the reaction was completed basically. The reaction solution was concentrated under reduced pressure, extracted with ethyl acetate, and washed with saturated brine, dried over anhydrous $Na_2SO_4$, and then filtered. The filtrate was concentrated under reduced pressure. The resulting crude product was dissolved in 2 ml of 1,4-dioxane and added with CDI (12 mg, 0.075 mmol). The reaction mixture was heated to reflux for 2 hours until the starting material was disappeared. 3 ml of 0.1 N sodium hydroxide aqueous solution was added, and the mixture was heated to 50 ° C and stirred for 1 hour. After cooling to room temperature, dilute hydrochloric acid was added to neutralize, and the mixture was extracted with ethyl acetate twice. The organic phase was washed with saturated brine and dried over anhydrous $Na_2SO_4$, and then filtered. The filtrate was concentrated under reduced pressure and the residues were purified by column chromatography to obtain compound **12**(15 mg, 65%).

$^1H$ NMR (400 MHz, $CDCl_3$) δ 10.53 (s, 1H), 3.63 (brs, 1H), 3.46 - 3.30 (m, 1H), 2.62 - 2.48 (m, 1H), 2.47 - 2.35 (m, 1H), 2.11 - 0.71 (m), 0.60 (s, 3H).

$^{13}C$ NMR (101 MHz, $CDCl_3$) δ 161.06, 159.69, 72.46, 70.93, 55.64, 50.58, 45.14, 42.83, 41.19, 40.03, 39.65, 35.51, 35.34, 33.83, 33.30, 31.94, 31.44, 30.38, 30.21, 29.69, 28.30, 23.69, 23.12, 22.24, 22.09, 20.75, 18.26, 11.80, 11.66.

**Example 13**

Preparation of 5-((3R)-3-((3R, 5S, 6R, 7R, 10S,13R)-6-ethyl-3,7-dihydroxy-10,13-dimethylhexadecahydro-1H-cyclopenta[a] phenanthrene-17-yl) butyl) -1,3,4-oxadiazole-2-carboxylic acid

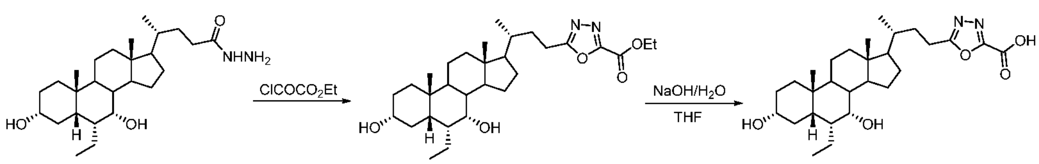


10-ii 13-ii 13

**Step 1:**

Methyl 6α-ethyl-chenodeoxycholicate **10-ii** (43 mg, 0.10 mmol) was dissolved in 2 mL of chloroform, and then added with pyridine (12 μL, 0.15 mmol, 1.5 eq.) and oxalyl chloride monoethyl ester (13 μL, 0.12 mmol, 1.2 eq.) successively. The reaction mixture was heated to 80 °C and stirred for 2 hours until the reaction was completed basically. The reaction solution was concentrated under reduced pressure, and then washed with saturated brine and extracted with ethyl acetate three times. The organic phases were combined and dried over anhydrous sodium sulfate, filtered, and filtrate was concentrated under reduced pressure. The residues were purified by column chromatography ($CH_2Cl_2$/MeOH 30:1) to obtain compound **13-ii** (22 mg, 43%).

$^1H$ NMR (400 MHz, $CDCl_3$) δ 4.31 (q, *J*= 7.1 Hz, 2H), 3.63 (brs, 1H), 3.40 -3.28 (m, 1H), 2.31 (ddd, *J*= 14.9, 10.3, 4.8 Hz, 1H), 2.16 (ddd, *J*= 14.7, 9.6, 4.7 Hz, 1H), 1.94 - 1.00 (m), 1.00 - 0.71 (m, H), 0.58 (s, 3H).

$^{13}C$ NMR (101 MHz, $CDCl_3$) δ 170.45, 158.61, 152.39, 72.34, 70.87, 63.61, 55.75, 50.54, 45.21, 42.77, 41.23, 40.06,

39.66, 35.51, 35.41, 33.90, 33.26, 31.34, 30.88, 30.59, 30.33, 28.23, 23.70, 23.15, 22.25, 20.77, 18.37, 13.97, 11.82, 11.67.

**Step 2:**

Compound **13-ii** (16 mg, 0.031 mmol) was dissolved in 1 mL of tetrahydrofuran, and then added with 1 mL of sodium hydroxide aqueous solution (0.5 N). The reaction mixture was heated to 60 °C and stirred for 2 hours. After completion of the reaction, the reaction solution was adjusted to acidity by addition of diluted hydrochloric acid, and then extracted with ethyl acetate. Compound **13** (14 mg, 93%)was obtained by column chromatography ($CH_2Cl_2$/MeOH 5:1).
$^1$H NMR (400 MHz, MeOD) δ 3.56 (brs, 1H), 2.31 - 2.17 (m, 1H), 2.17 - 2.03 (m, 1H), 1.99 - 0.73 (m), 0.61 (s, 3H).
$^{13}$C NMR (101 MHz, MeOD) δ 173.80, 160.17, 157.52, 71.81, 69.82, 55.97, 50.28, 45.57, 42.38, 41.77, 40.18, 39.67, 35.40, 35.25, 33.15, 33.05, 31.39, 30.36, 29.87, 27.87, 23.18, 22.37, 22.10, 20.59, 17.50, 10.88, 10.64.

**Example 14**

Preparation of 5-((2R)-2-((3R, 5S, 6R, 7R, 10S, 13R)-6-ethyl-3,7-dihydroxy-10,13-dimethylhexadecahydro-1H-cyclopenta[a] phenanthrene-17-yl)propyl) thiazolidine-2,4-dione

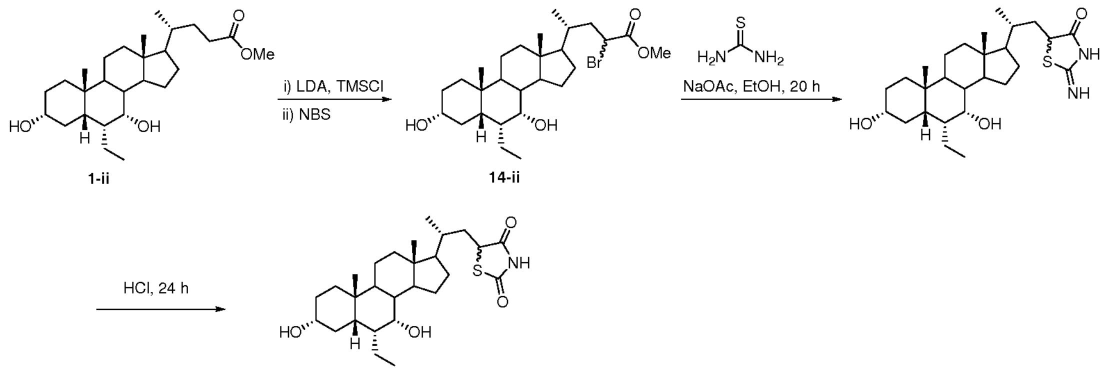


1-ii

14-ii

14-α, 14-β

**Step 1:**

Methyl 6α-ethyl-chenodeoxycholicate **1-ii** (845 mg, 1.94 mmoL) was dissolved in 10 mL of dry tetrahydrofuran, and the mixture was cooled to -72 °C in an ice-ethanol bath. LDA solution (4.9 mL, 9.72 mmol, 2 M) was added dropwise over 15 minutes and further stirred for 15 min before addition of trimethylchlorosilane (1.0 mL, 11.7 mmol) dropwise. The reaction mixture was stirred for 4 hours at low temperature. N-bromosuccinimide (692 mg, 3.89 mmol) was dissolved in 5 ml of tetrahydrofuran and then added dropwise to the reaction system. After completion of the addition, the reaction mixture was warmed to room temperature and stirred overnight. After TLC showed completion of the reaction, the reaction solution was quenched with 10 ml of water, and extracted with ethyl acetate three times. The organic phases were combined, washed with saturated brine, dried over anhydrous sodium sulfate, and filtered. The filtrate was concentrated under reduced pressure to obtain crude product **14-ii** which was used directly in the next step.

**Step 2:**

Compound **14-ii** was dissolved in 20 ml of ethanol, and then added with thiourea (443 mg, 5.82 mmol) and sodium acetate (477 mg, 5.82 mmol). The mixture was heated to reflux and stirred overnight. After cooling to room temperature, 2N hydrochloric acid (20 mL) was added and the reaction was continued under reflux for 24 hours. The reaction solution was cooled, and concentrated under reduced pressure to remove ethanol. The residues were extracted with ethyl acetate three times. The organic phases were combined, dried over anhydrous sodium sulfate, filtered and the filtrate was concentrated under reduced pressure. The crude product was purified by column chromatography to obtain a mixture of diastereoisomers 14-α and 14-β (487 mg, 52%).
**14-α:** $^1$H NMR (400 MHz, MeOD) δ 4.13 (dd, *J*= 7.4, 5.8 Hz, 1H), 3.60 (brs, 1H), 3.31 - 3.20 (m, 1H), 2.30 (ddd, *J*= 14.3, 5.6, 3.6 Hz, 1H), 1.96 - 1.02 (m), 1.02 - 0.75 (m, 12H), 0.61 (s, 3H).
$^{13}$C NMR (101 MHz, MeOD) δ 177.04, 172.50, 71.95, 70.57, 56.15, 50.36, 49.34, 45.24, 42.90, 41.33, 40.17, 39.91,

39.56, 35.48, 35.44, 35.22, 33.32, 33.14, 30.17, 28.36, 23.53, 23.06, 22.10, 20.71, 18.76, 11.60, 11.46.
**14-β:** $^{1}H$ NMR (400 MHz, $CDCl_3$) δ 4.31 (dd, *J*= 11.3, 3.3 Hz, 1H), 3.70 (brs, 1H), 3.53 - 3.37 (m, 1H), 2.02 -1.08 (m), 1.08 - 0.78 (m, 12H), 0.67 (s, 3H).
$^{13}C$ NMR (101 MHz, $CDCl_3$) δ 176.05, 171.36, 72.51, 71.02, 56.25, 50.49, 50.09, 45.10, 42.90, 41.24, 40.20, 39.97, 39.57, 35.54, 35.51, 35.45, 33.60, 33.08, 30.27, 28.39, 23.70, 23.09, 22.21, 20.72, 17.58, 11.81, 11.65.

**Example 15**

Preparation of 5-((2R)-2-((3R,5S,7R, 10S,13R)-3,7-dihydroxy-10,13-dimethylhexadecahydro-1H-cyclopenta[a]phenanthrene-17-yl)propyl)thiazolidine-2,4-dione

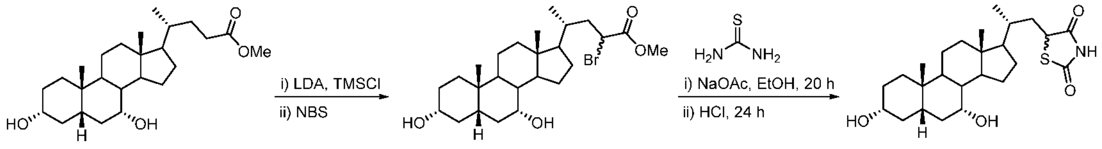


15-i 15-ii 15-α , 15-β

**Step 1:**

Methyl chenodeoxycholicate **15-i** (348 mg, 0.86 mmoL) was dissolved in 5 mL of dry tetrahydrofuran and the mixture was cooled to -72 °C in an ice-ethanol bath. LDA solution (2.1 mL, 4.28 mmol, 2M) was added dropwise in 15 minutes and further stirred for 15min before addition of trimethylchlorosilane (0.44 mL, 5.14 mmol) dropwise . The reaction mixture was stirred for 4 hours at low temperature. N-bromosuccinimide (305 mg, 1.71 mmol) was dissolved in 5 ml of tetrahydrofuran and then added dropwise to the reaction system above. After completion of the addition, the reaction was warmed to room temperature and then stirred overnight. After TLC showed completion of the reaction, the reaction solution was quenched with 10 ml of water, and extracted with ethyl acetate three times. The organic phases were combined, washed with saturated brine, dried over anhydrous sodium sulfate, and filtered. The filtrate was concentrated under reduced pressure to obtain crude product **15-ii** which was used directly in the next step.

**Step 2:**

Compound **15-ii** was dissolved in 10 ml of ethanol, and then added with thiourea (196 mg, 2.58 mmol) and sodium acetate(222 mg, 2.58 mmol). The mixture was heated to reflux and stirred for 6 hours. After cooling to room temperature, 2N hydrochloric acid (10 mL) was added and the reaction was continued under reflux overnight. The reaction solution was cooled to room temperature and concentrated under reduced pressure to remove ethanol, and then extracted with ethyl acetate three times. The organic phases were combined, dried over anhydrous sodium sulfate, filtered and the filtrate was concentrated under reduced pressure. The crude product was purified by column chromatography to obtain a mixture of diastereoisomers 15-α and 15-β (220 mg, 57%).
**15**-α: $^{1}H$ NMR (400 MHz, $CDCl_3$) δ 8.27 (s, 1H), 4.16 (dd, *J*= 7.1, 6.1 Hz, 1H), 3.79 (d, *J*= 2.3 Hz, 1H), 3.48 - 3.35 (m, 1H), 2.32 (ddd, *J*= 14.3, 5.8, 3.6 Hz, 1H), 2.13 (dd, *J*= 24.6, 12.8 Hz, 1H), 1.96 -1.02 (m), 1.02 - 0.73 (m), 0.62 (s, 3H).
$^{13}C$ NMR (101 MHz, $CDCl_3$) δ 175.14, 170.32, 72.01, 68.54, 56.15, 50.45, 49.20, 42.99, 41.47, 40.25, 39.89, 39.64, 39.41, 35.33, 35.25, 35.05, 34.75, 32.87, 30.67, 28.42, 23.74, 22.75, 20.59, 18.89, 11.76.
**15**-β: $^{1}H$ NMR (400 MHz, $CDCl_3$) δ 9.87 (s, 1H), 4.23 (dd, *J*= 11.5, 3.8 Hz, 1H), 3.79 (brs, 1H), 3.53 - 3.28 (m, 1H), 2.32 - 0.98 (m), 0.98 - 0.70 (m), 0.60 (s, 3H).
$^{13}C$ NMR (101 MHz, $CDCl_3$) δ 176.19, 171.57, 72.12, 68.62, 56.15, 50.33, 50.09, 42.81, 41.42, 40.26, 39.56, 39.31, 35.45, 35.33, 35.06, 34.78, 32.70, 30.37, 29.69, 28.41, 23.67, 22.74, 20.57, 17.58, 11.78.

**Biological Experiment Example 1**

**FXR receptor binding activity assay (time-resolved fluorescence detection method TR-FRET method)**

To test the effect of the present compounds on FXR receptor binding, we selected the commercial FXR receptor binding activity assay kit to test the effect of the compounds on FXR and its binding protein fragments (LBD structural domain binds to SRC1 fragment) (kit supplier: Life Technology Invitrogen Catalog: PV4835; cisbio Catalog: 610SAXLA and 61GSTKLA). Specific method was described in the kit instructions. The operation was as follows:

1. Preparing 2x GST-labeled FXR-LBD/anti-GST Eu mixture to the required volume, the concentration of GST-FXR-LBD was 6nM, Eu was 50nl/well.
2. Preparing 2x biotin-labeled SRC1/streptomycin-allophycocyanin(SA-APC) mixture to the required volume, the concentration of SRC1 was 1000 nM, and streptomycin-allophycocyanin was 50 nl/well.
3. Mixing 2x GST-FXR/Eu and SRC1/SA-APC in the proportion of 1:1.
4. 20 μl of a mixture of GST-FXR/Eu and SRC1/SA-APC was added to a 384-well plate containing the compounds to be tested.
5. Centrifugation for 1min at 1000 rpm; incubating for 180min at room temperature.
6. Time-resolved fluorescence detection: EnVision reader.
**7.The analysis results:**

(1) the value at 665nm divided by the value at 615nm;
(2) calculating the agonist rate

$$\text{Agonist rate} = (\text{X-Min}) / (\text{Max-Min}) * 100\%$$

- X represents the 665/615 value for each concentration;
- Min represents the 665/615 value without adding compound;
- Max represents the 665/615 value of the reference compound.

The biochemical activity of the present compounds was determined by the above test, and the $EC_{50}$ values measured were shown in the table below.

| Number of examples | $EC_{50}$(uM) |
|---|---|
| 1 | 0.214 |
| 2 | 0.167 |
| 3 | 0.016 |
| 4 | 0.054 |
| 5 | 0.031 |
| 6 | 1.267 |
| 7 | 0.552 |
| 8 | 0.228 |
| 9 | 0.432 |
| 10 | 0.061 |
| 11 | 0.366 |
| 12 | 0.052 |
| 13-ii | 0.070 |
| 13 | 0.283 |
| 14-α | 0.013 |
| 14-β | 0.004 |
| 15-α | 0.143 |
| 15-β | 0.312 |

Conclusion: the example compounds of the present invention had a significant agonist effect on FXR activity.

**Biological Experiment Example 2**

**FXR reporter gene expression test**

To analyze the effect of testing compounds on FXR-regulated gene expression, we used Promega's dual luciferase assay system (Promega #E1980) to detect the effect of the compounds on the FXR reporter gene in HepG2 hepatoma cell line (ATCC #HB-8065) which transiently was transfected by the FXR Gal4 reporter gene fragment. Specific experimental methods are as follows:

**1. HepG2 cell culture and maintenance**

Cells were digested and cells with appropriate density were grown in 10 ml complete medium and then cultivated for 24 hours under 37 °C 5% $CO_2$ humidity conditions.

**2. Cell planting and transfection.** Transfection reagent was Fugene HD (Promega#E231A)

(1) Preparing the transfection mixture according to the following table

| | |
|---|---|
| pBIND-FXR(ng/well) | 25 |
| pG5Luc(ng/well) | 25 |
| FuGENE HD(μl/well) | 0.15 |
| Serum-free medium (μl/well) | 1.85 |
| Total mixture (μl/well) | 2.5 |

(2) The mixture in the tube was vigorously mixed and incubated at room temperature for 15 minutes;
(3) Digesting the cells in the culture dish and counting;
(4) Diluting the cell suspension to the desired volume of 600,000/ml (96-well plates 100μl /well);
(5) Adding the required volume of the transfection mixture to the cell suspension, followed plating at 100 μl/well;
(6) Incubateing for 24 hours under 37 °C 5% $CO_2$ humid conditions.

**3. Treatment of the compound**

(1) 10 mM of mother liquor of the compound was prepared and then serially diluted with DMSO by 3 times;
(2) 10 μl of compound was added to 90 μl of complete medium (10x);
(3) 5 μl of the solution of the compound (21x) was added to each well;
(4) Incubating for 18 hours under 37 °C 5% $CO_2$ humidity conditions.

**4. Dual-luciferase assay**

The firefly luciferase and the renilla luciferase signal (Promega #E1980) were detected by Promega's dual-luciferase assay system.

**5. The analysis results**

(1) The detected values were given according to Firefly luciferase signal (F) divided by the renilla luciferase signal(R), F/R. This data processing eliminated the difference resulted from cell number and transfection efficiency.
(2) Calculating the agonist rate

$$\text{Agonist rate} = (\text{X-Min}) / (\text{Max-Min}) * 100\%$$

- X represents the F/R value of each concentration;
- Min represents the F/R value without adding compound;
- Max represents the F/R value of the reference compound.

(3) Calculate $EC_{50}$ according to GraphPrism 5.0

The biochemical activity of the present compounds was determined by the above test, and the $EC_{50}$ values measured were shown in the table below.

| Number of examples | $EC_{50}$(uM) |
|---|---|
| 1 | 2.6 |
| 2 | 3.388 |
| 3 | 2.529 |
| 4 | 1.064 |
| 8 | 3.373 |
| 10 | 1.399 |
| 11 | 5.200 |
| 12 | 1.364 |
| 13 | 5.199 |
| 14-α | 0.063 |
| 14-β | 0.057 |
| 15-β | 3.37 |

Conclusion: the example compounds of the present invention had a significant agonist effect on FXR activity.

**Biological Experiment Example 3**

**(Pharmacokinetic test in rats)**

The pharmacokinetic study of the present invention was performed in rats. The pharmacokinetic behavior of the positive control INT-747 and the representative compound of the present invention such as Example 14-β in rats was studied, and their pharmacokinetic characteristics were evaluated.

**1. Experimental protocol**

(1) Three male SD rats were used , which were supplied by Sippr-BK laboratory animal Co. Ltd. Animal Production License No. SCXK (Shanghai) 2008-0016.
(2) 10 mg of the sample was weighed and added with 10 ml of 0.5% CMC-Na aqueous solution, the mixture was fully shakened uniform to make 1 mg/ml of solution, the remaining liquid samples was for quantitative analysis.
(3) Three male SD rats. were intragastrically administrated at 10 mg/kg with the volume of 10 ml/kg after fasting for one night.
(4) About 0.05 ml of underjaw intravenous blood was collected before and 0.5, 1.0, 2.0, 4.0, 6.0, 8.0, 24.0, 30.0 h after administration and then placed in a EDTA-K2 test tube. The blood was centrifuged at 6000 rpm for 5 minutes to isolate the plasma which was stored at -80 °C. The rats were feeded 4 h after administration.

**2. Analytical methods**

(1) Instruments and Equipment: API 4000 triple quadrupole mass spectrometer, Applied Biosystems, USA; Shimadzu LC-20AD high performance liquid chromatography system, Shimadzu, Japan.
(2) Condition of chromatography: The chromatographic column was Phenomenex gemiu 5um C18 50X4.6 mm; mobile phase was acetonitrile: 0.1% TFA aqueous solution (gradient elution); flow rate (ml/min) was 0.8 ml/min.
(3) Condition of mass spectrometry:

| | |
|---|---|
| Potential of entrance | 10 psi |
| Potential of collision cell export | 12 psi |

(continued)

| | |
|---|---|
| Curtain gas | 15 psi |
| Collision Gas | medium |
| Scanning mode | ESI(+), MRM |
| Ionspray voltage | 5500 V |
| Temperature | 550 °C |
| Ion source gas 1 | 50 psi |
| Ion source gas 2 | 50 psi |

**3. Pharmacokinetic test results:**

(1) The results showed that the peak time of blood concentration $t_{max}$ is 0.5h, the peak concentration $C_{max}$ is 4413ng/ml, the area under the blood concentration-time curve $AUC_{0-t}$ is 9028 ng/ml • h after intragastric administration of 10mg/kg of positive control INT-747.
(2) The results of the test showed that the peak time of blood concentration $t_{max}$ is 1 h, the peak concentration $C_{max}$ is 7577 ng/ml, the area under the blood concentration-time curve $AUC_{0-t}$ is 41796 ng/ml•h after the intragastric administration of 10mg/kg of compound of example 14-β.

Pharmacokinetic test in rats shows that the exposed quantity of the present compound of example 14-βin the tested animals is significantly better than that of the positive control INT-747.

## Claims

**1.** A cholic acid derivative of formula (I), or a stereoisomer or a pharmaceutically acceptable salt thereof:

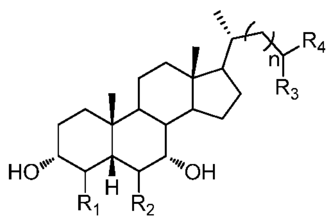

(I)

wherein,

$R_1$, $R_2$ and $R_3$ are each selected from the group consisting of hydrogen, fluorine, bromine, trifluoromethyl and $C_{1-8}$ alkyl;
$R_4$ is selected from the group consisting of:

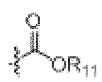 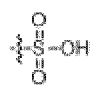 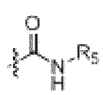 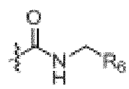

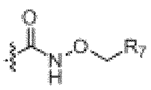 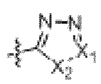 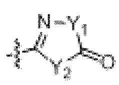 and 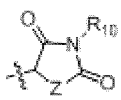 ;

$X_1$ is selected from the group consisting of nitrogen and $CR_8$;
$X_2$ is selected from the group consisting of $CR_8R_9$, $NR_{10}$, oxygen and sulfur atom;
$Y_1$ and $Y_2$ are each independently selected from the group consisting of $CR_8R_9$, $NR_{10}$, oxygen and sulfur atom;
Z is selected from the group consisting of $CR_8R_9$, $NR_{10}$, oxygen and sulfur atom;
$R_5$ is selected from the group consisting of hydrogen, hydroxy, cyano, amino, $C_{1-8}$ alkoxy and $SO_2R_{11}$;
$R_6$ is selected from the group consisting of $SO_2R_{11}$, $SO_3R_{11}$, $C(O)OR_{11}$, $C(O)R_{12}$ and $C_{0-4}$-$P(O)R_{13}R_{14}$;
$R_7$ is selected from the group consisting of hydroxy, hydroxyl substituted $C_{1-8}$ alkyl and halo$C_{1-8}$ alkyl;
$R_8$ and $R_9$ are each independently selected from the group consisting of hydrogen, deuterium, halogen, hydroxy, cyano, nitro, $C_{1-8}$ alkyl, $C_{2-8}$ alkenyl, $C_{2-8}$ alkynyl, $C_{3-8}$ cycloalkyl, $C_{1-8}$ alkoxy, $C_{3-8}$ cycloalkoxy, $SO_2R_{11}$, $C(O)OR_{11}$, $C(O)R_{12}$ and $P(O)R_{13}R_{14}$, wherein the alkyl and cycloalkyl are each optionally substituted by one or more groups selected from the group consisting of halogen, hydroxy, $C_{1-8}$ alkyl, $C_{1-8}$ alkoxy, halo$C_{1-8}$ alkoxy, $C_{3-8}$ cycloalkyl and $C_{3-8}$ cycloalkoxy;
$R_{10}$ is selected from the group consisting of hydrogen, $C_{1-8}$ alkyl, $C_{3-8}$ cycloalkyl, halo $C_{1-8}$ alkyl, and $C_{1-8}$ alkoxy;
$R_{11}$ is selected from the group consisting of hydrogen, $C_{1-8}$ alkyl, $C_{3-8}$ cycloalkyl, halo $C_{1-8}$ alkyl, phenyl and p-methylphenyl;
$R_{12}$, $R_{13}$ and $R_{14}$ are each independently selected from the group consisting of hydroxy, $C_{1-8}$ alkyl, $C_{3-8}$ cycloalkyl, halo$C_{1-8}$-alkyl, $C_{1-8}$alkoxy, amino, and $C_{1-8}$alkyl disubstituted amino;
n is 0 or 1.

**2.** The cholic acid derivatives, or a stereoisomer or a pharmaceutically acceptable salt thereof according to claim 1, wherein $C_{1-8}$ alkyl is preferred selected from $C_{1-6}$ alkyl, and more preferred selected from $C_{1-3}$ alkyl; $C_{1-8}$ alkoxy is preferred selected from $C_{1-6}$ alkoxy, and more preferred selected from $C_{1-3}$ alkoxy; $C_{3-8}$ cycloalkyl is preferred selected from $C_{3-6}$ cycloalkyl; $C_{3-8}$ cycloalkoxy is preferred selected from $C_{3-6}$ cycloalkoxy; $C_{2-8}$ alkenyl is preferred selected from $C_{2-4}$ alkenyl; $C_{2-8}$ alkynyl is preferred selected from $C_{2-4}$ alkynyl.

**3.** The cholic acid derivatives, or a stereoisomer or a pharmaceutically acceptable salt thereof according to claim 1, wherein $R_3$ is selected from the group consisting of hydrogen, methyl, ethyl and isopropyl; $R_1$, $R_2$, $R_4$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$, Z and n are as defined in claim 1.

**4.** The cholic acid derivatives, or a stereoisomer or a pharmaceutically acceptable salt thereof according to claim 1, wherein $R_3$ is selected from the group consisting of hydrogen and methyl; $R_1$, $R_2$, $R_4$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$, Z and n are as defined in claim 1.

**5.** The cholic acid derivatives, or a stereoisomer or a pharmaceutically acceptable salt thereof according to claim 1, wherein the compound is a compound of formula (II):

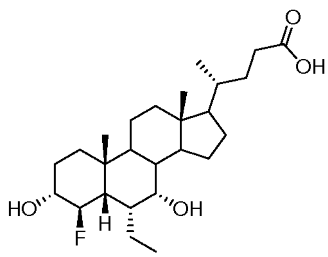

(II)

.

**6.** The cholic acid derivatives, or a stereoisomer or a pharmaceutically acceptable salt thereof according to claim 1, wherein $R_1$ is hydrogen; $R_3$ is selected from the group consisting of hydrogen and methyl; $R_2$, $R_4$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$, Z and n are as defined in claim 1.

**7.** The cholic acid derivatives, or a stereoisomer or a pharmaceutically acceptable salt thereof according to claim 1, wherein the compound is a compound of formula (III):

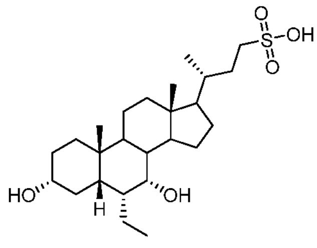

(III)

.

8. The cholic acid derivatives, or a stereoisomer or a pharmaceutically acceptable salt thereof according to claim 1, wherein the compound is a compound of formula (IV)

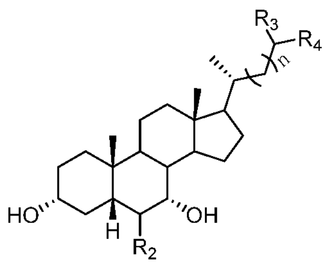

(IV)

wherein, $R_4$ is selected from the group consisting of:

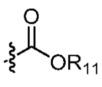 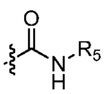 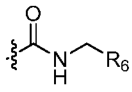 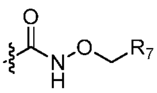 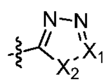 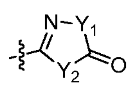 and 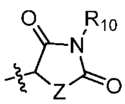 ;

$R_2$, $R_3$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$, Z and n are as defined in claim 1.

9. The cholic acid derivatives, or a stereoisomer or a pharmaceutically acceptable salt thereof according to claim 8, wherein when $R_4$ is selected from the group consisting of

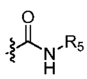 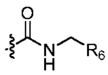 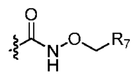 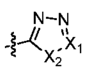 and 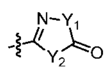 ,

n is 1;
when $R_4$ is

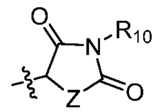 ,

n is 0;
$R_2$, $R_3$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$ and Z are as defined in claim 1.

10. The cholic acid derivatives, or a stereoisomer or a pharmaceutically acceptable salt thereof according to claim 8, wherein the compound is selected from the group consisting of:

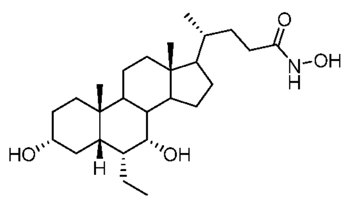
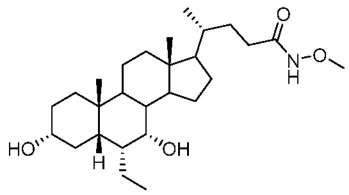
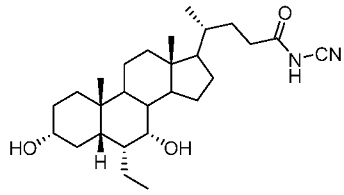

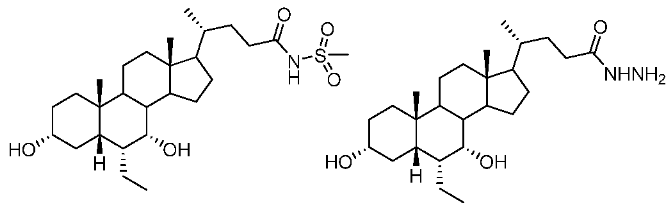
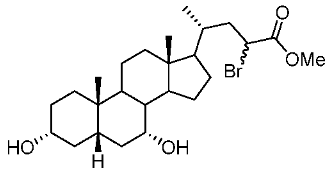

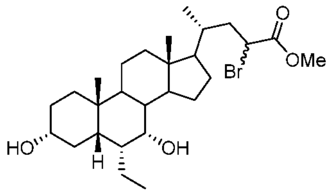

11. The cholic acid derivatives, or a stereoisomer or a pharmaceutically acceptable salt thereof according to claim 8, wherein the compound is selected from the group consisting of:

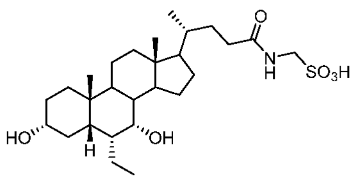
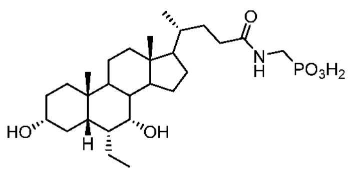
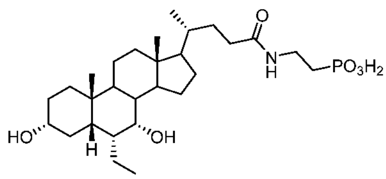

.

12. The cholic acid derivatives, or a stereoisomer or a pharmaceutically acceptable salt thereof according to claim 8, wherein the compound is:

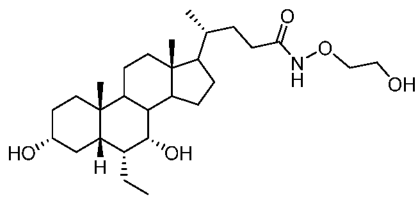

.

13. The cholic acid derivatives, or a stereoisomer or a pharmaceutically acceptable salt thereof according to claim 8, wherein the compound is a compound of formula (V):

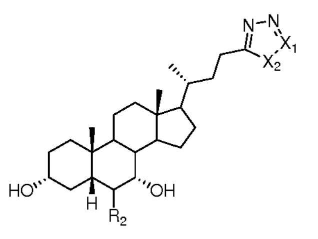

(V)

wherein, $X_1$, $X_2$, $R_2$, $R_8$, $R_9$, $R_{10}$, $R_{11}$, $R_{12}$, $R_{13}$ and $R_{14}$ are as defined in claim 1.

**14.** The cholic acid derivatives, or a stereoisomer or a pharmaceutically acceptable salt thereof according to claim 8, wherein the compound is selected from the group consisting of:

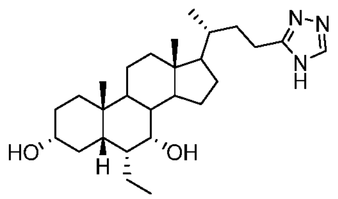

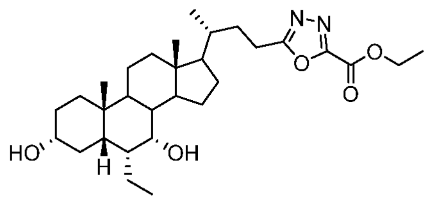

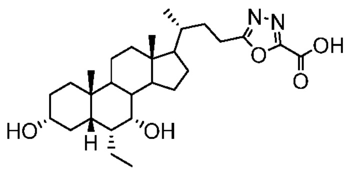

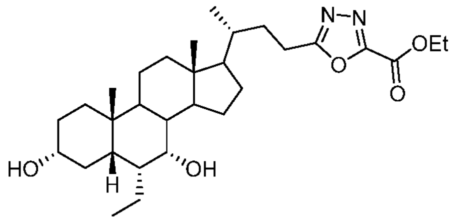

.

**15.** The cholic acid derivatives, or a stereoisomer or a pharmaceutically acceptable salt thereof according to claim 8, wherein the compound is a compound of formula (VI):

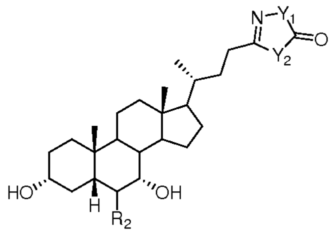

(VI)

wherein, $Y_1$, $Y_2$, $R_2$, $R_8$, $R_9$, $R_{10}$, $R_{11}$, $R_{12}$, $R_{13}$ and $R_{14}$ are as defined in claim 1.

**16.** The cholic acid derivatives, or a stereoisomer or a pharmaceutically acceptable salt thereof according to claim 8, wherein the compound is selected from the group consisting of:

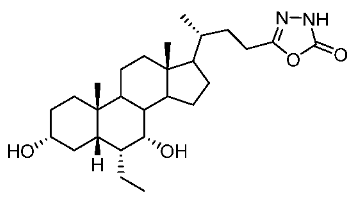

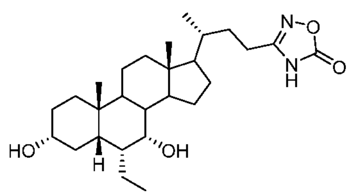

.

**17.** The cholic acid derivatives, or a stereoisomer or a pharmaceutically acceptable salt thereof according to claim 8, wherein the compound is a compound of formula (VII):

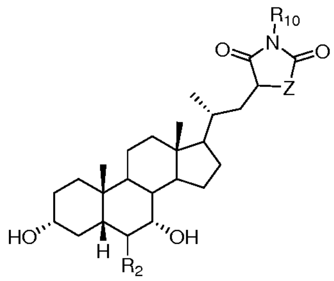

(VII)

wherein, $R_2$, $R_8$, $R_9$, $R_{10}$, $R_{11}$, $R_{12}$, $R_{13}$, $R_{14}$ and Z are as defined in claim 1.

**18.** The cholic acid derivatives, or a stereoisomer or a pharmaceutically acceptable salt thereof according to claim 8, wherein the compound is selected from the group consisting of:

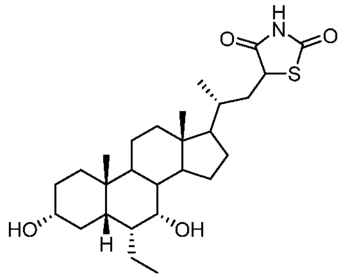
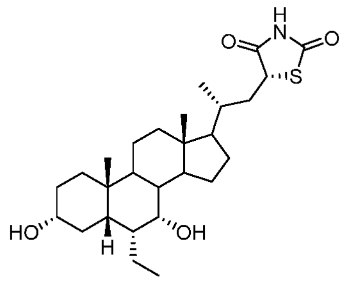
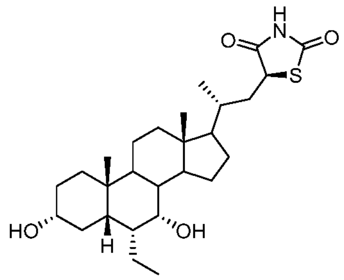

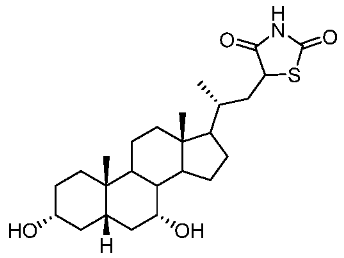
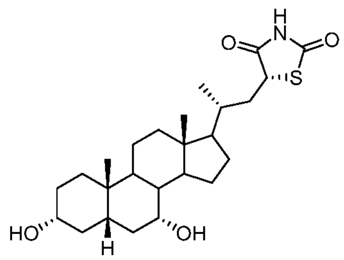
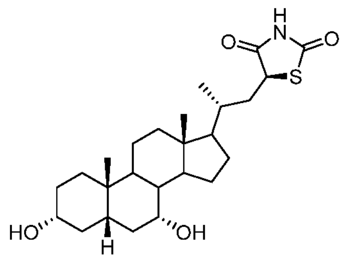
.

**19.** A cholic acid derivative of formula (VIII), or a stereoisomer or a pharmaceutically acceptable salt thereof:

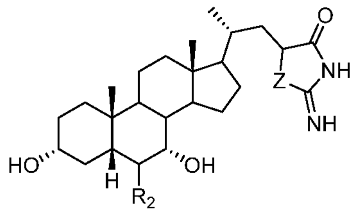

(VIII)

Z is selected from the group consisting of oxygen and sulfur stom;
$R_2$ is selected from the group consisting of hydrogen, fluorine, bromine, trifluoromethyl and $C_{1-8}$ alkyl.

**20.** The cholic acid derivatives, or a stereoisomer or a pharmaceutically acceptable salt thereof according to claim 19, wherein the compound is selected from the group consisting of:

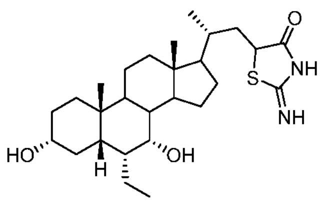
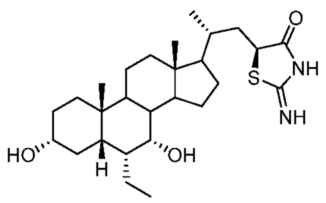
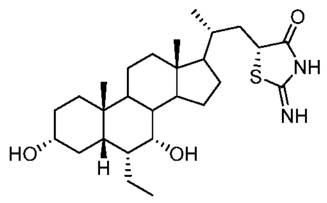

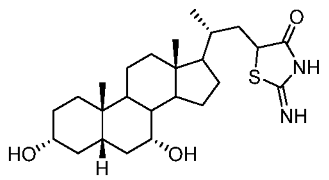
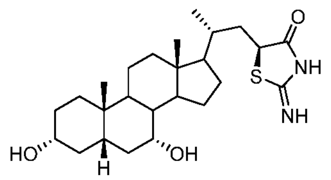
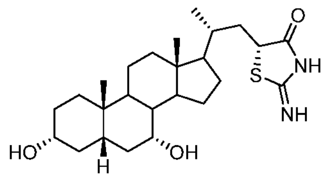
.

21. A process for preparing the cholic acid derivatives, or a stereoisomer or a pharmaceutically acceptable salt thereof according to any one of claims 1 to 20, comprising the steps of:

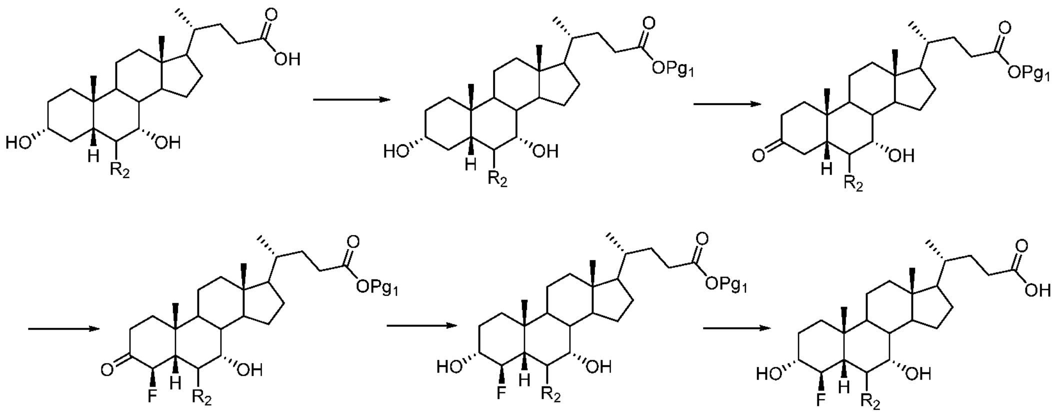

or

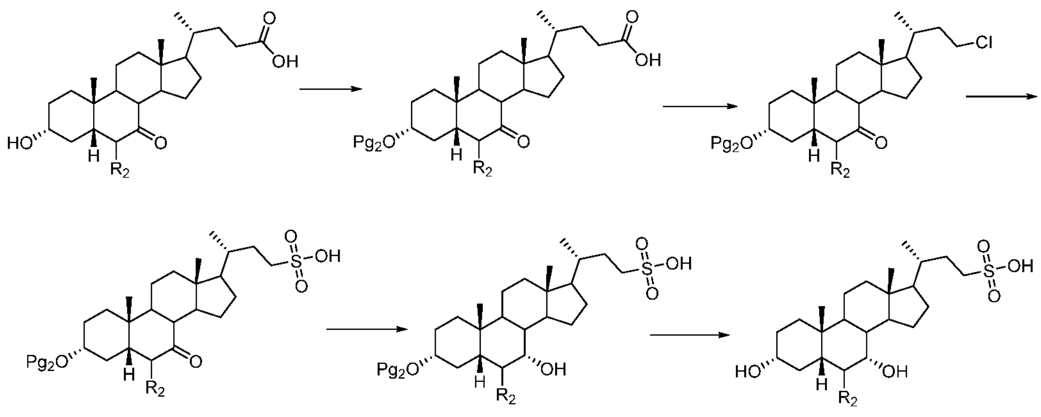

or

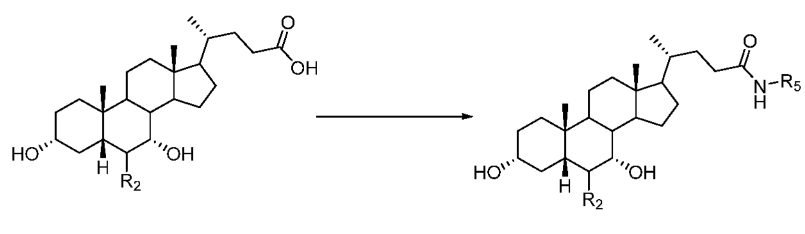

or

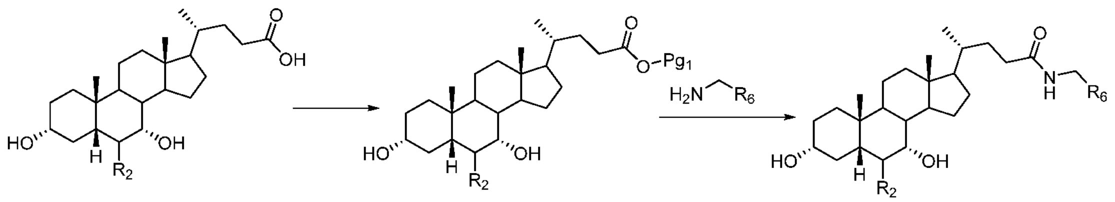

or

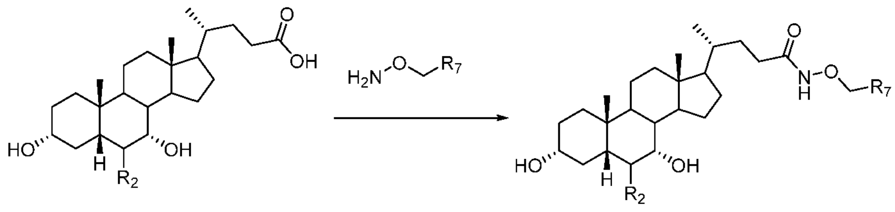

or

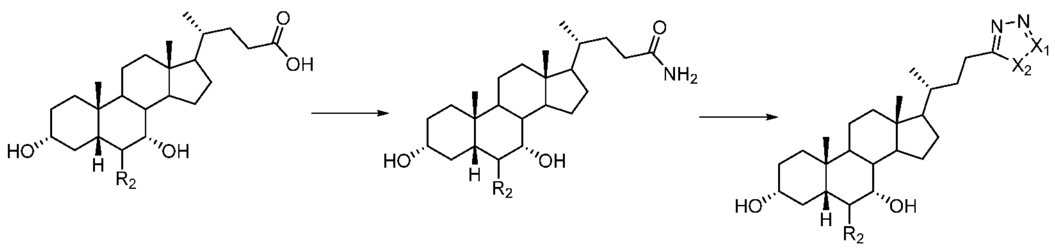

or

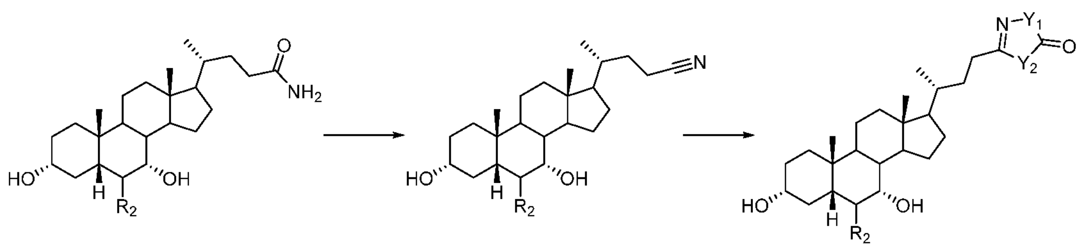

or

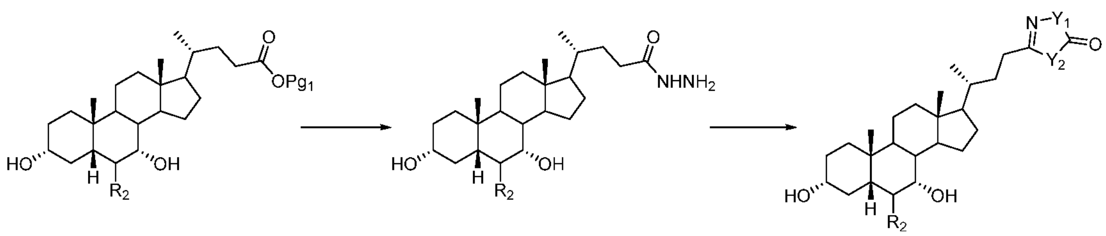

or

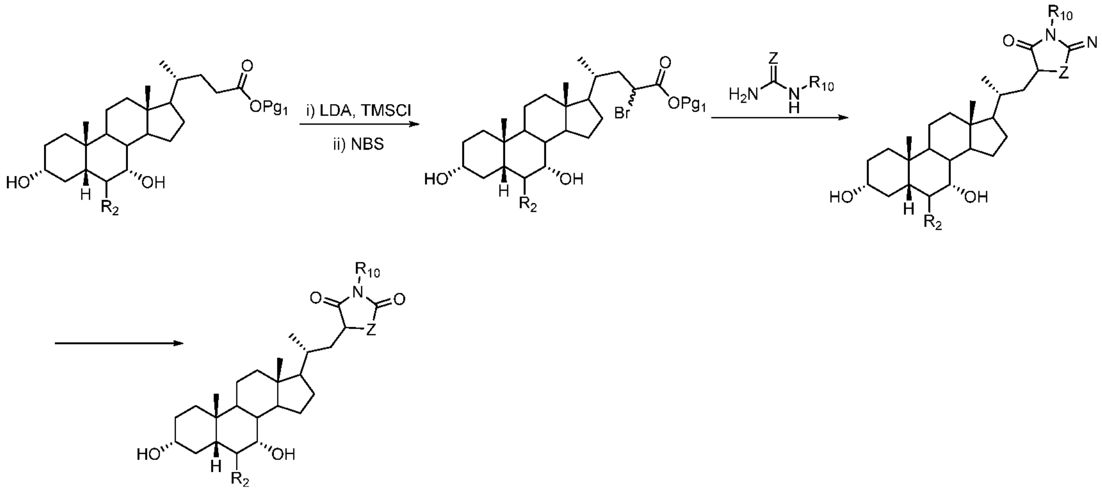


wherein, $R_2$, $R_5$, $R_6$, $R_7$, $R_8$, $R_9$, $R_{10}$, $R_{11}$, $R_{12}$, $R_{13}$, $R_{14}$, $X_1$, $X_2$, $Y_1$, $Y_2$ and Z are as defined in claim 1; $Pg_1$ is hydroxyl protective group, and it is preferred selected from the group consisting of $C_{1-4}$ alkyl and benzyl, $Pg_2$ is hydroxyl protective group, and it is preferred selected from the group consisting of $C_{1-4}$ alkyl and acetyl.

**22.** A pharmaceutical composition comprising a therapeutically effective amount of the cholic acid derivatives, or a stereoisomer or a pharmaceutically acceptable salt thereof according to any one of claims 1 to 20, and a pharmaceutical acceptable carrier.

**23.** Use of the cholic acid derivatives, or a stereoisomer or a pharmaceutically acceptable salt thereof according to any one of claims 1 to 20, or the pharmaceutical composition according to claim 22, in the preparation of medicament for the prevention or treatment of FXR mediated diseases and conditions.

**24.** The use according claim 23, wherein the FXR mediated diseases and conditions are selected from the group consisting of cardiovascular disease, hypercholesterolemia, hyperlipidemia and chronic hepatitis disease, chronic liver disease, gastrointestinal disease, nephrosis, cerebrovascular disease, metabolic disease and cancer.

**25.** The use according claim 24, wherein chronic liver disease is selected from the group consisting of primary cirrhosis (PBC), Cerebrotendinous Xanthomatosis (CTX), primary sclerosing cholecystitis (PSC), cholestasis caused by drug, intrahepatic cholestasis of pregnancy, parenteral nutrition associated cholestasis (PNAC), cholestasis of bacterial overgrowth or pyemia, autoimmune hepatitis, chronic viral hepatitis, alcoholic liver disease, non-alcoholic fatty liver disease (NAFLD), non-alcoholic steatohepatitis (NASH), graft versus host disease related to liver transplantation, regeneration of living donor liver transplantation, congenital hepatic fibrosis, choledocholithiasis, granulation liver disease, intrahepatic and extrahepatic malignant tumor, Sjogren syndrome, sarcoidosis, Wilson's disease, Gaucher's disease, hemochromatosis and $\alpha$1-antitrypsin deficiency; gastrointestinal disease which is selected from the group consisting of inflammatory bowel disease (IBD), irritable bowel syndrome (IBS), bacterial overgrowth, nutrient malabsorption, colonitis after reflection and microscopic colitis, inflammatory bowel disease which is preferred selected the group consisting of Crohn's Disease and ulcerative enteritis; nephrosis which is selected from the group consisting of diabetic nephropathy, focal segmental glomerulosclerosis (FSGS), hypertensive nephropathy, chronic glomerulitis, chronic transplant glomerulopathy, chronic interstitial nephritis and polycystic kidney disease; cardiovascular disease which is selected from the group consisting of arteriosclerosis, arteriosclerosis, atherosclerosis, dyslipidemia, hypercholesterolemia and hypertriglyceridemia; metabolic disease which is selected from the group consisting of insulin resistance, type I diabetes, type II diabetes and obesity; cerebrovascular disease which is selected from stroke; cancer which is selected from the group consisting of colorectal cancer and liver cancer.

**26.** A method to prevente and treat FXR mediated diseases and conditions, comprising administration of a therapeutically effective amount of the cholic acid derivatives, or a stereoisomer or a pharmaceutically acceptable salt according to any one of claims 1 to 20, or the pharmaceutical composition according to claim 22.

# INTERNATIONAL SEARCH REPORT

International application No.
PCT/CN2016/079167

**A. CLASSIFICATION OF SUBJECT MATTER**

C07J 9/00 (2006.01) i; C07J 31/00 (2006.01) i; C07J 41/00 (2006.01) i; C07J 51/00 (2006.01) i; C07J 43/00 (2006.01) i; C07J 75/00 (2006.01) i; A61K 31/58 (2006.01) i; A61K 31/575 (2006.01) i; A61P 9/00 (2006.01) i; A61P 3/06 (2006.01) i; A61P 1/16 (2006.01) i; A61P 1/00 (2006.01) i

According to International Patent Classification (IPC) or to both national classification and IPC

**B. FIELDS SEARCHED**

Minimum documentation searched (classification system followed by classification symbols)

C07J 9/-

Documentation searched other than minimum documentation to the extent that such documents are included in the fields searched

Electronic data base consulted during the international search (name of data base and, where practicable, search terms used)

CNABS, CNKI, WPI, EPODOC, REGISTRY, CAPLUS: bile acid, farnesoid X receptor, derivative, farnesoid derivative X receptor, FXR, substructure search according to I-VII formular

**C. DOCUMENTS CONSIDERED TO BE RELEVANT**

| Category* | Citation of document, with indication, where appropriate, of the relevant passages | Relevant to claim No. |
|---|---|---|
| X | NGUYEN, T.T.H. et al. "Synthesis of 24-(piperidin-1-yl, morpholin-4-yl and 4-methylpiperazin-1-yl)-5β-cholan-3α-ols and four hydroxylated 23-(4,5-dihydroimidazol-2-yl)-24-nor--5β-cholanes", COLLECTION OF CZECHOSLOVAK CHEMICAL COMMUNICATIONS, vol. 62, no. 3, 31 December 1997 (31.12.1997), pages 471-478, and see page 472, abstract | 1-26 |
| X | D'AMORE, C. et al. "Design, Synthesis, and Biological Evaluation of Potent Dual Agonists of Nuclear and Membrane Bile Acid Receptors", JOURNAL OF MEDICINAL CHEMISTRY, vol. 57, no. 3, 04 January 2014 (04.01.2014), pages 937-954, and see page 938, Fig.1 | 1-26 |
| X | HONORIO, K.M. et al. "Hologram QSAR studies on farnesoid X receptor activators", LETTERS IN DRUG DESIGN & DISCOVERY, vol. 3, no. 4, 31 December 2006 (31.12.2006), pages 261-267, and see pages 262-264, Table 1 | 1-26 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 30 June 2016 (30.06.2016) | **20 July 2016 (20.07.2016)** |
| Name and mailing address of the ISA/CN:<br>State Intellectual Property Office of the P. R. China<br>No. 6, Xitucheng Road, Jimenqiao<br>Haidian District, Beijing 100088, China<br>Facsimile No.: (86-10) 62019451 | Authorized officer<br>**WANG, Shaohua**<br>Telephone No.: (86-10) **62086353** |

Form PCT/ISA/210 (second sheet) (July 2009)

# INTERNATIONAL SEARCH REPORT

International application No.
PCT/CN2016/079167

C (Continuation). DOCUMENTS CONSIDERED TO BE RELEVANT

| Category* | Citation of document, with indication, where appropriate, of the relevant passages | Relevant to claim No. |
|---|---|---|
| X | US 5466815 A (HOECHST AG), 14 November 1995 (14.11.1995), see col. 22, example 9 | 1-26 |
| X | WO 2015017813 A3 (US HEALTH et al.), 02 April 2015 (02.04.2015), see claim 31 | 1-26 |
| X | US 2014206657 A1 (HOPE CITY), 24 July 2014 (24.07.2014), see claim 21 | 1-26 |
| PX | CN 104672290 A (BEIJING PRELUDE PHARMACEUTICAL TECHNOLOGY CO., LTD.), 03 June 2015 (03.06.2015), see claim 5 | 1-26 |

Form PCT/ISA/210 (continuation of second sheet) (July 2009)

## INTERNATIONAL SEARCH REPORT

International application No.
**PCT/CN2016/079167**

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 26
because they relate to subject matter not required to be searched by this Authority, namely:
[1] claim 26 relates to a precaution or treatment method performed on human or animal bodies (PCT Rules 39.1 (iv)). Nonetheless, search is performed based on a technical subject about the use of said compounds/compositions in preparing corresponding drugs.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet (2)) (July 2009)

**INTERNATIONAL SEARCH REPORT**
Information on patent family members

International application No.
**PCT/CN2016/079167**

| Patent Documents referred in the Report | Publication Date | Patent Family | Publication Date |
|---|---|---|---|
| US 5466815 A | 14 November 1995 | GR 3026496 T3 | 31 July 1998 |
| | | AT 163938 T | 15 March 1998 |
| | | ES 2115096 T3 | 16 June 1998 |
| | | IL 109581 D0 | 26 August 1994 |
| | | CA 2123048 C | 29 November 2005 |
| | | CZ 289515 B6 | 13 February 2002 |
| | | DK 0624596 T3 | 07 October 1998 |
| | | IL 109581 A | 10 March 1998 |
| | | CA 2123048 A1 | 09 November 1994 |
| | | EP 0624596 B1 | 11 March 1998 |
| | | FI 942075 A | 09 November 1994 |
| | | TW 289757 B | 01 November 1996 |
| | | JP H06329695 A | 29 November 1994 |
| | | NZ 260469 A | 28 March 1995 |
| | | EP 0624596 A3 | 07 June 1995 |
| | | AU 6194794 A | 10 November 1994 |
| | | DE 59405410 D1 | 16 April 1998 |
| | | NO 941677 A | 09 November 1994 |
| | | NO 304795 B1 | 15 February 1999 |
| | | EP 0624596 A2 | 17 November 1994 |
| | | CY 2119 B1 | 26 April 2002 |
| | | HU 217439 B | 28 January 2000 |
| | | NO 941677 D0 | 06 May 1994 |
| | | FI 942075 A0 | 05 May 1994 |
| | | HU T67390 A | 28 April 1995 |
| | | HU 9401443 D0 | 29 August 1994 |
| | | CZ 9401135 A3 | 15 December 1994 |
| | | AU 666440 B2 | 08 February 1996 |
| | | JP 3476157 B2 | 10 December 2003 |
| WO 2015017813 A3 | 02 April 2015 | CA 2920017 A1 | 05 February 2015 |
| | | AU 2014296023 A1 | 25 February 2016 |
| | | CN 105593237 A | 18 May 2016 |
| | | WO 2015017813 A2 | 05 February 2015 |
| US 2014206657 A1 | 24 July 2014 | None | |
| CN 104672290 A | 03 June 2015 | None | |

Form PCT/ISA/210 (patent family annex) (July 2009)